# EUROPEAN PATENT APPLICATION

(11) **EP 2 832 234 A1**
(43) Date of publication of application: **04.02.2015**
(21) Application number: 13178628.7
(22) Date of filing: 30.07.2013
(51) Int. Cl.: A23L 1/22, A23L 1/226, C07D 471/04

(54) **Imidazo[1,2-a]pyridine-ylmethyl-derivatives and their use as flavoring agents**

(71) Applicant: IMAX Discovery GmbH, 44227 Dortmund (DE)
(72) Inventor: WINNING, Marcel, 20091 BRESSO (MI) (IT); LOHMER, Stefan, 20091 BRESSO (MI) (IT); PEVARELLO, Paolo, 20091 BRESSO (MI) (IT)
(74) Representative: Banfi, Paolo

(57) **Abstract**

The present invention primarily relates to imidazo[1,2-a]pyridine-ylmethyl-derivatives of Formula (I) wherein R¹, R², X, W e J are as defined in the description, to mixtures thereof and to the use thereof as flavoring agents. The compounds in accordance with the present invention are suitable for producing, imparting, or intensifying an umami flavor. The invention further relates to flavoring mixtures, compositions for oral consumption as well as ready-to-eat, ready-to-use and semifinished products, comprising an effective amount of the compound of Formula (I) and to specific methods for producing, imparting, modifying and/or intensifying specific flavor impressions.

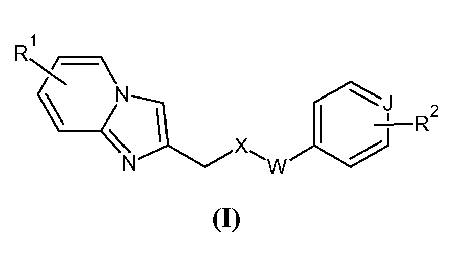

## Description

### Field of the invention

The present invention primarily relates imidazo[1,2-a]pyridine-ylmethyl-derivatives and to the use thereof as flavoring agents. The compounds in accordance with the present invention are suitable for producing, imparting, or intensifying an umami flavor. The invention further relates to flavoring mixtures, compositions for oral consumption as well as ready-to-eat, ready-to-use and semifinished products, comprising an effective amount of the compounds of Formula (I) and to specific methods for producing, imparting, modifying and/or intensifying specific flavor impressions.

### Background of the invention

One of the basic tastes is the "umami" flavor, produced upon oral consumption of monosodium glutamate (sodium glutamate, "MSG"). MSG is known to produce adverse reactions in some people, and some progress has been made in identifying artificial substitutes for MSG. It is known that a few naturally occurring materials can increase or enhance the effectiveness of MSG as a savory flavoring agent, so that less MSG would be needed for a given flavoring application. For example the naturally occurring nucleotide compounds inosine monophosphate (IMP) or guanosine monophosphate (GMP) are known to have a multiplier effect on the savory taste of MSG, but IMP and GMP are very difficult and expensive to isolate and purify from natural sources, or synthesize, and hence have only limited practical application to most commercial applications. New flavoring agents able to provide a taste profile similar to that of MSG, in order to partially or fully substitute MSG in a flavor, or new compounds that enhance the effectiveness of MSG in order to substitute IMP or GMP as MSG enhancers, could be of high value.

In the past years some alternative substances have been described as being able to create or enhance umami taste, in particular for replacing monosodium glutamate. Some of these "umami"-compounds have already been awarded GRAS (generally recognized as safe) status by the FEMA for use in food products.

US 2009/0311401 and US 2009/0110796 propose certain neomenthyl derivatives as flavor materials mainly having umami and/or salty taste. US 2009/0311401 inter alia describes the flavor profile of the racemate of (1S,2S,5R)-2-isopropyl-5-methylcyclohexyl ethyl carbamate as "cooling, umami, menthol note".

US 2009/0280230 proposes the use of certain savory taste modifiers as flavoring agents and/or enhancers of monosodium glutamate in a process for preparing a solid flavorant concentrate composition or a lipophilic savory flavorant concentrate composition.

US 2011/0020517 suggests certain pyridine-derivatives to impart or enhance a kokumi or umami taste.

WO 2013000673 A1 and US 20120308703 A1 describe certain cinnamamides able to confer, enhance, improve o modify the kokumi or umami taste.

Compounds enhancing the umami taste are also disclosed in WO2005041684 A1 and WO20070124152 A1.

There remains in the art a need for new and preferably improved flavoring agents, i.e. flavor-active compounds or compounds which can produce, impart, modify or intensify a flavor impression. In particular, there is a demand for compounds of the type that can produce, impart, modify or intensify the "umami" impression. In particular, flavoring agents providing a complex and multi-facetted taste profile, e.g. one or more further flavor aspects in addition to an umami taste, are of particular interest. In combination with other flavoring agents, preferably of certain taste directions, such flavoring agents would allow the creation of interesting sensorial impressions and exceptional taste profiles.

The object of the present invention is therefore to provide one or more compounds and corresponding methods and uses by means of which certain flavor notes, preferably of the "umami" type, can be produced, imparted, modified or intensified (enhanced).

Compounds of the umami type may well be combined with other sensorially active substances imparting one or more flavor impressions such as warming, hot, tingling, numbing, sulfurous, alliaceous, phenolic, smoky, tangy (sharp) and/or pungent flavor notes. Although such flavor notes are often characteristic for a certain flavor direction, these notes are often experienced as unpleasant, objectionable and/or disturbing, in particular as too strong or too dominant within a flavor profile. However, without these flavor notes or without the substances producing these flavor notes, the flavor direction would be experienced as less typical, less authentic, less valuable, and often would be less preferred by consumers.

It would thus additionally be desirable if the one or more new compounds would be able not only to produce or enhance certain desired notes, but also reduce one or more unwanted notes, improve and/or round off the overall flavor profile without impairing or adulterating it to an appreciable extent.

In particular, said one or more new compounds should be able to enhance, intensify and/or round off one or more sensory impressions of one or more other flavoring substances, wherein preferably one, several or all of the sensory impressions mouthfeel, meaty, roasty, salty, mouth-watering and long-lastingness of said or more other flavoring substances are enhanced, intensified, and/or rounded off.

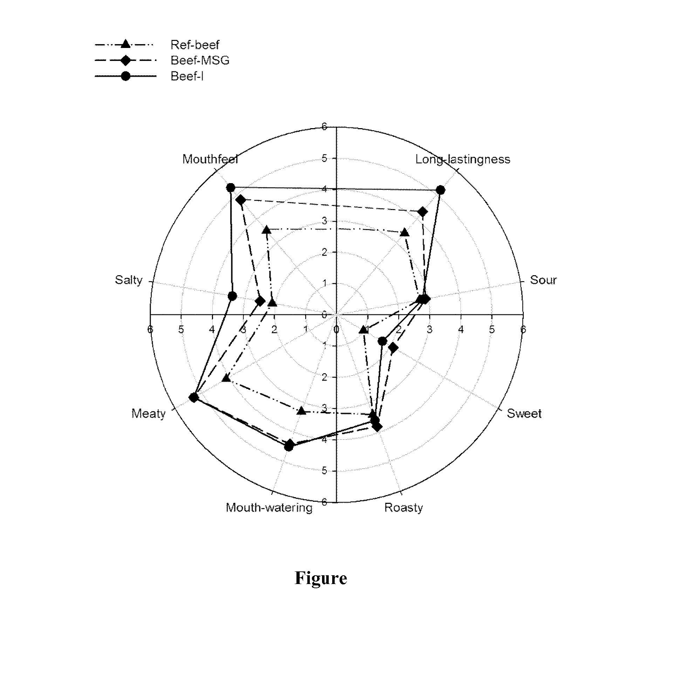

### Description of the figure

**FIGURE**: Panelists' scores of strength / intensity of eight different flavour descriptors.

### Brief description of the invention

According to the present invention, the object set forth above is achieved by using a compound of Formula (I) 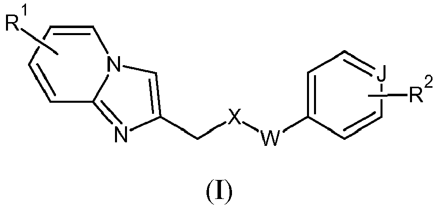 wherein:
X is O or NR³, wherein R³ is hydrogen or C₁-C₄ alkyl;
W is a carbonyl or a sulfonyl group, with the proviso that when W is a sulfonyl group, then X is NR³;
J is CH or N;
R¹ is hydrogen, halogen or C₁-C₄ alkyl;
R² is hydrogen, halogen, C₁-C₄ alkyl, C₁-C₄ alkoxy, C₁-C₄ thioalkyl, sulfonamide, NR³R⁴, wherein R³ and R⁴ are, independently from each other, hydrogen or C₁-C₄ alkyl, OCH₂R⁵, wherein R⁵ is an aromatic or heterocyclic ring;
or R² is COR⁶, wherein R⁶ is C₁-C₄ alkyl, aryl C₁-C₄ alkyl, or a phenyl ring optionally substituted with C₁-C₄ alkyl, C₁-C₄ alkoxy or halogen groups;
mixtures thereof and salts thereof suitable for human or animal consumption;
as food additive, beverage additive, and/or flavoring agent.

### Detailed description of the invention

In a preferred embodiment this object is achieved by a compound of Formula (I),
wherein:
X is O or NR³ wherein R³ is methyl;
W is a carbonyl or a sulfonyl group;
J is CH or N;
R¹ is halogen;
R² is halogen, C₁-C₄ alkyl, C₁-C₄ alkoxy, C₁-C₄ thioalkyl, OCH₂R⁵, wherein R⁵ is phenyl or a five-membered heterocyclic ring containing at least one oxygen atom;
or R² is COR⁶, wherein R⁶ is C₁-C₄ alkyl or a phenyl ring substituted with one C₁-C₄ alkyl group.
In a further preferred embodiment this object is achieved by a compound of Formula (I),
wherein:

X is O or NR³ wherein R³ is methyl;
W is a carbonyl or a sulfonyl group;
J is CH or N;
R¹ is chlorine;
R² is C₁-C₄ thioalkyl, OCH₂R⁵, wherein R⁵ is phenyl or tetrahydrofuranyl ring;
or R² is COR⁶, wherein R⁶ is methyl or a phenyl ring substituted with a methyl group.

In a further preferred embodiment this object is achieved by a compound of Formula (I),
selected from:
- imidazo[1,2-a]pyridine-2-ylmethyl 4-(oxolan-2-ylmethoxy)benzoate, having CAS Registry Number 1090752-50-3 and formula: 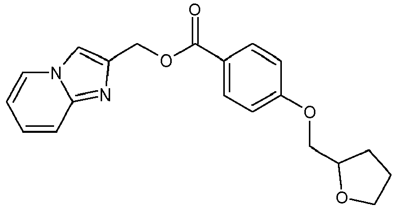
- 6-chloroimidazo[1,2-a]pyridine-2-ylmethyl 2-(4-methylbenzoyl)benzoate, having CAS Registry Number 1012292-27-1 and formula: 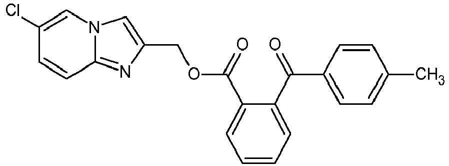
- imidazo[1,2-a]pyridine-2-ylmethyl 2-(methylsulfanyl)pyridine-4-carboxylate, having CAS Registry Number 1089612-41-8 and formula: 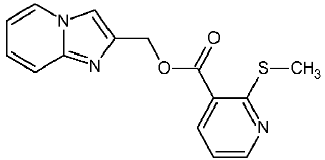
- 4-acetyl-N-((6-chloroimidazo[1,2-a]pyridine-2-yl)methyl)-N-methylbenzene-1-sulfonamide having CAS Registry Number: 1090444-32-8 and formula:

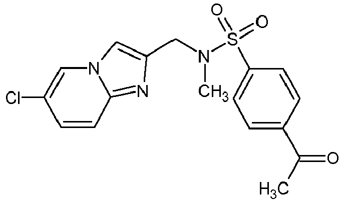

The present invention also relates to a mixture of compounds comprising, essentially consisting of or consisting of compounds of Formula (I).

The compounds of Formula (I) and the mixtures thereof were found to have interesting sensorial properties allowing to produce, impart, modify or intensify an "umami" impression.

In combination with other flavoring agents, preferably of certain taste directions indicated below, the compounds of Formula (I) and the mixtures thereof allow the creation of interesting sensorial impressions and exceptional taste profiles.

The compounds of Formula (I) and the mixtures thereof are able to enhance, intensify, round off, harmonize, balance, smooth and/or mellow one or more sensory impressions of one or more other flavoring substances, preferably of one or more other flavoring substances producing or imparting one, several or all of the sensory impressions mouthfeel (sometimes also called "fullness in the mouth"), meaty, roasty, salty, mouth-watering and long-lastingness.

In particular, in combination with other flavoring substances producing a warming, hot, tingling, numbing, sulfurous, alliaceous, phenolic, smoky, tangy and/or pungent (piquant) flavor impression, the compounds of Formula (I) and the mixtures thereof are able to produce a flavor profile that is perceived as smoother, mellower, rounder (more rounded-off), more harmonious, more balanced and more pleasant, thus as having overall improved flavor properties.

In another aspect, the present invention therefore relates to the use of a
(i) compound of Formula (I),
(ii) mixture of compounds of Formula (I),
   or
(iii) a composition comprising one or more compounds of Formula (I),
as food additive, beverage additive, and/or flavoring agent.

Our own sensory tests revealed that the compounds of Formula (I) according to the present invention or the mixtures thereof can impart or modify the above mentioned sensory effects in MSG-reduced or in MSG-free orally consumable compositions.

The initial flavor impact, the flavor profile and the longer-lastingness - and thus also the overall sensory experience - was generally felt to be pleasant and multifaceted, in many cases even preferred and more varied in comparison to corresponding flavor containing MSG instead of the compounds of Formula (I).

The compounds of the Formula (I) and the mixtures thereof produce an umami taste and intensify or enhance the umami taste of flavored compositions. In our own investigations a trained panel found, in a statistically significant manner, that simply by adding 10 ppm of a compound of formula (I) to a bouillon of 0.5 wt.% of beef extract, the umami-effect observed was roughly the same as is achieved by the addition of 0.05 wt.% (500 ppm) of MSG.

The flavor impressions mouthfeel, salty, meaty, roasty, sweet, sour, mouth-watering and long-lastingness were intensified by the addition of the compounds of the Formula (I), in some cases they were even stronger enhanced when using the compounds of the Formula (I) as compared to using MSG (see Sensory Test 2 described in detail hereinbelow).

In a preferred embodiment, the present invention relates to the use of a compound of Formula (I), or a composition comprising one or more compounds of Formula (I), as flavoring agent for
(i) producing, imparting, or intensifying an umami flavor and/or a physiological cooling effect,
   and/or
(ii) enhancing, intensifying and/or rounding off one or more sensory impressions of one or more other flavoring substances [i.e. flavoring substances not corresponding to Formula (I) or not comprising a compound of Formula (I)], wherein preferably one, several or all of the sensory impressions mouthfeel, meaty, roasty, salty, mouth-watering and long-lastingness of said or more other flavoring substances are enhanced, intensified, and/or rounded off (i.e. the flavor profile and overall taste is harmonized and smoothed, e.g. the sensory impression is improved because being less sharp, less pungent, more natural, and more balanced).

In a preferred aspect, the present invention relates to the use of a compound of Formula (I), or a composition comprising one or more compounds of Formula (I), for producing, imparting, modifying or intensifying an umami flavor, preferably an umami flavor.

It was further found in the course of our own investigations that the above described sensory effects and the prominent performance were particularly and in a more pronounced manner observed in liquid compositions. For example, when the compounds of Formula (I) were evaluated as part of a solid food product (e.g. solid snacks like crackers, chips, nuts, beef jerky and the like), the sensory effects described above were somewhat weaker in comparison to the same concentration of the compounds of Formula (I) in a non-solid food product (e.g. wet soups, spreads, spicy sauces, creams, pastes, and the like).

In the context of the present invention, the term "flavor" includes both gustatory and nasal sensory impressions, perceptions and effects. Thus, the term "flavor" includes the taste perceived on the tongue and the smell (aroma, bouquet) perceived in the nose (via orthonasal and/or retronasal perception). However, regarding the sensory effects umami, mouth-watering, and mouthfeel (and preferably also physiological cooling), the term "flavor" is understood as relating to taste impressions, perceptions and effects, since said sensory effects mainly or solely occur in the mouth and/or on the tongue.

In the context of the present invention, the term "sensorially effective amount" defines an amount sufficient to produce a flavor impression upon oral consumption, in particular an umami and/or a physiological cooling impression, and/or to modify (including intensifying, enhancing, rounding off, reducing unwanted notes, and the like) the perception of a flavor of one or more other flavoring agents, in particular other flavoring agents producing or imparting one, several, or all of the following aspects or sensory effects mouthfeel, meaty, roasty, salty, mouth-watering and long-lastingness.

In the context of the present invention, "essentially consists of" or "essentially consisting of" means that the total weight share is 92 wt.% or more, preferably 95 wt.% or more, more preferably 96 wt.% or more, most preferably 97 wt.% or more, based on the total respective mass used.

The compounds of Formula (I) may be obtained in analogy to well-known synthetic methods and some of them are also commercially available.

For example, the compounds of the invention of Formula (I) wherein W is a carbonyl group can be obtained by reacting a compound of Formula (II) 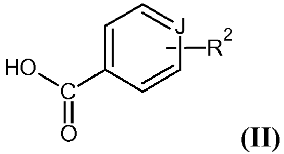 wherein J and R² are as defined in Formula (I), with a suitable activating agent of the carboxylic acid group, for example with an acylating agent able to form a mixed anhydride between the compound of formula (II) and the acylating agent, preferably with trifluoroacetic anhydride, in an organic solvent, preferably toluene, at a suitable temperature, preferably at room temperature, for a certain time, preferably between one and eighteen hours, followed by reaction with a compound of formula (III) 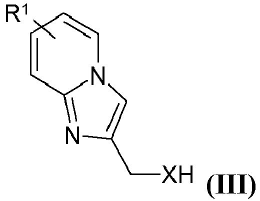 wherein R¹ and X are as defined in Formula (I), for a certain time, preferably between one and eighteen hours.

Alternatively, a compound of formul (II) can be converted in the corresponding acyl chloride and reacted with a compound of formula (III) under the standard conditions used for the formation of ester or amido bonds.

Alternatively, the compounds of formula (II) may be reacted with a compound of formula (IV) 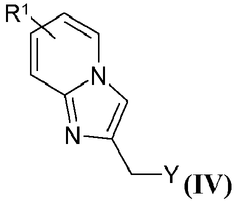 wherein R¹ is as defined in formula (I) and Y is a suitable leaving group, such as an halide group or triflate group, preferably a bromine group or a triflate group, in an organic solvent, preferably toluene, or N,N-dimethylformamide, or dimethylsulphoxide, in the presence of an organic or inorganic base, at a suitable temperature, preferably at room temperature, for a certain time, preferably between one and eighteen hours.

Other compounds of the invention of Formula (I) wherein W is a sulfonyl group and Z is NR³ wherein R³ is as defined in Formula (I) can be obtained by reacting a compound of Formula (V) 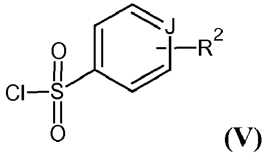 wherein J and R² are as defined in Formula (I), with a compound of Formula (VI), 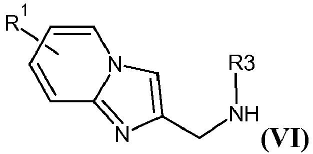 wherein R¹ and R³ are as defined in Formula (I), in the presence of a base, such as triethylamine or dimethylaminopyridine (DMAP), preferably triethylamine, in an organic solvent such as dichloromethane or dimethylformamide, preferably dichloromethane, at a suitable temperature, between 0°C and room temperature, preferably at room temperature, for a certain time, preferably between one and eighteen hours.

According to a further aspect, the present invention relates to a flavoring mixture comprising
(A) a sensorially effective amount of a compound of Formula (I) or of a mixture of the compounds of Formula (I),
   and
(B) a sensorially effective amount of one or more, preferably two or more, more preferably three or more other ingredients [i.e. additional ingredients not corresponding to Formula (I) selected from the group of substances imparting one or more sensory (preferably one or more flavor) impressions, preferably substances imparting one or more sensory impressions selected from the group consisting of warming, heating, tingling, numbing, stinging, salivating (i.e. salivation-inducing substances), sulfurous, alliaceous (i.e. substances part of the aroma or reminiscent of *Allium* species), pungent (piquant), meat, fish, seafood, seaweed, mushroom, aromatic, smoky, tangy, spicy, herbal, savory, and mouthfeel.

Preferably, a flavoring mixture according to the present invention, comprises one or more, more preferably two or more, even more preferably three or more additional ingredients of group (B) are selected from the group consisting of constituents (B-1), (B-2), (B-3), (B-4), (B-5), (B-6), and (B-7)

(B-1) substances selected from the group consisting of physiologically warming and/or tingling sensates,

(B-2) aroma molecules imparting one or more sulfurous, alliaceous and/or pungent notes, preferably selected from the group consisting of notes associated with radish, horseradish, wasabi, mustard, onion, garlic, leek, shallot, chive, and cress,

preferably selected from the group consisting of sulfur-containing aroma molecules with a molecular weight of less than 300 g/mol, preferably selected from the group consisting of thiols (mercaptans), thioethers, disulfides, trisulfides, thiophenes, thiocyanates, isothiocyanates, thionoesters, thioesters, dithiines, dithianes, dithiazines, pentathiepanes, tetrathianes, dithiolanes, trithiolanes, and S-oxides, more preferably with a molecular weight of less than 290 g/mol, more preferably with a molecular weight of less than 270 g/mol, particularly preferably with a molecular weight of less than 250 g/mol, most preferably with a molecular weight of less than 235 g/mol,

(B-3) aroma molecules found in meat or contributing to the aroma of meat, preferably selected from the group consisting of

C4-C12-pyrazines (i.e. pyrazines having a total of 4 to 12 carbon atoms), C5-C12-pyridines, C4-C12-pyrroles, C4-C12-pyrrolines, C4-C10-oxazolines C4-C10-thiazoles, C4-C10-thiazolines, C3-C8-thiazolidines, C4-C12-furans, C4-C12-furanones, straight- or branched-chain alkanals having a total of 4 to 15 carbon atoms, straight- or branched-chain alkanols having a total of 5 to 10 carbon atoms, C4-C15-ketones, gamma-lactones having an even-number of total carbon atoms in the range of 6 to 12 carbon atoms, delta-lactones having an uneven-number of total carbon atoms in the range of 7 to 13 carbon atoms, C4-C8-alkanoic acids, C6-C 15-hydrocarbons, and C16-C30-alkanoic acids having 0 to 5 (i.e. 0, 1, 2, 3, 4 or 5) carbon-carbon-double bonds, and C6-C12-phenols with 1 or 2 hydroxyl groups and 0 to 2 (i.e. 0, 1 or 2) methoxy groups,

(B-4) aroma molecules imparting one or more sensorial impressions selected from the group consisting of thyme, clove, cinnamon, pimento, black pepper, white pepper, red chili, green chili, anise, lemongrass, vanilla, coconut, caramel, coriander, ginger, basil, sweet basil, tarragon, lemon, curry, fenugreek, and lime,

(B-5) amino acids, preferably selected from the group consisting of amino carboxylic acids and amino sulfonic acids, and the physiologically acceptable salts thereof,

(B-6) further flavor enhancers for meaty and/or roasty notes,
and

(B-7) substances selected from the group consisting of beef extract, soy sauce, soy sauce extract, soy protein, yeast extract, autolyzed yeast extract, malt extract, hydrolyzed vegetable protein, whey protein, caseinate, and barley extract, preferably in dry (i.e. essentially water-free, preferably water-free), more preferably in powdered form.

The agents of constituent (B1) - upon oral consumption - produce a perception of a physiological warming, hot, burning and/or tingling sensation to the consumer, and may additionally be accompanied by a numbing and/or pungent sensation.

The aroma molecules of constituent (B3) contribute to the flavor profiles of cooked and/or smoked meat, in particular boiled, roasted, grilled, fried or smoked meat, in particular boiled, roasted, grilled, fried or smoked beef. The sensory impression "meat" preferably relates to cooked meat. Based on sensory evaluation, several different qualities or descriptors are generally used to describe the flavor of cooked meat: buttery, caramel, burnt/roasty, green, oily/fatty, nutty and meaty. Constituent (B3) also includes aroma molecules typically found in smoke (flavor), e.g. obtained by pyrolysis of wood.

A preferred flavoring mixture according to the present invention comprises one or more, preferably two or more, more preferably three or more additional ingredients selected from the group consisting of constituents (B-1), (B-2), (B-3), (B-4), (B-5), (B-6), and (B-7), wherein constituent

(B-1) comprises or consists of substances selected from the group consisting of spilanthol (2E,6Z,8E-decatrienoic acid N-isobutylamide), 2E,6Z,8E,10E-dodecatetraenoic acid N-(2-methylpropyl)amide ([alpha]-sanshool), 2E,6Z,8E,10E-dodecatetraenoic acid N-(2-hydroxy-2-methylpropyl)amide [alpha]-hydroxysanshool), 2E,6E,8E,10E-dodecatetraenoic acid N-(2-hydroxy-2-methylpropyl)amide ([gamma]-hydroxysanshool), 2E,4E,8Z,10E,12E-tetradecapentaenoic acid N-(2-hydroxy-2-methylpropyl)amide ([gamma]-hydroxysanshool), 2E,4E,8E,10E,12E-tetradecapentaenoic acid N-(2-hydroxy-2-methylpropyl)amide ([gamma]-hydroxyisosanshool), 2E,4E,8Z,10E,12E-tetradecapentaenoic acid N-(2-methyl-2-propenyl)amide ([gamma]-dehydrosanshool), 2E,4E,8Z,10E,12E-tetradecapentaenoic acid N-(2-methylpropyl)amide ([gamma]-sanshool), 2E,4E,8Z,11Z-tetradecatetraenoic acid N-(2-hydroxy-2-methylpropyl)amide (bungeanool), 2E,4E,8Z,11E-tetradecatetraenoic acid N-(2-hydroxy-2-methylpropyl)amide (isobungeanool), 2E,4E,8Z-tetradecatrienoic acid N-(2-hydroxy-2-methylpropyl)amide (dihydrobungeanool), 2E,4E-tetradecadienoic acid N-(2-hydroxy-2-methylpropyl)amide (tetrahydrobungeanool), 2E,4E-decadienoic acid N-isobutylamide (trans-pellitorine), 2E,4Z-decadienoic acid N-isobutylamide (cis-pellitorine), 2Z,4Z-decadienoic acid N-isobutylamide, 2Z,4E-decadienoic acid N-isobutylamide, 2E,4E-decadienoic acid N-([2S]-2-methylbutyl)amide, 2E,4E-decadienoic acid N-([2S]-2-methylbutyl)amide, 2E,4E-decadienoic acid N-([2R]-2-methylbutylamide), 2E,4Z-decadienoic acid N-(2-methylbutyl)amide, 2E,4E-decadienoic acid N-piperide (achilleamide), 2E,4E-decadienoic acid N-piperide (sarmentine), 2E-decenoic acid N-isobutylamide, 3E-decenoic acid N-isobutylamide, 3E-nonenoic acid N-isobutylamide, 2E,7Z,9E-undeca-2,7,9-trienoic acid N-isobutyl amide, 2E-undeca-2-en-8,10-diynoic acid N- isobutyl amide,

vanillyl alcohol n-propyl ether, vanillyl alcohol isopropyl ether, vanillyl alcohol n-butyl ether, vanillyl alcohol isobutyl ether, vanillyl alcohol isoamyl ether, vanillyl alcohol n-hexyl ether vanillyl alcohol methyl ether, vanillyl alcohol ethyl ether, gingerols (preferably 6-gingerol, 8-gingerol, 10-gingerol), gingerdiones (preferably gingerdione-[2], gingerdione-[3], gingerdione-[4], gingerdione-[6], gingerdione-[8], gingerdione-[10], gingerdione-[12]), shogaols (preferably [6]-shogaol, [8]-shogaol, [10]-shogaol), paradols (preferably [6]-paradol, [8]-paradol, [10]-paradol), zingerone, capsaicin, dihydrocapsaicin, nordihydrocapsaicin, homocapsaicin, homodihydrocapsaicin, pseudocapsaicin (nonivamide; N-[(4-hydroxy-3-methoxyphenyl)methyl]nonanamide),

chavicine, piperine, 1'-acetoxychavicol, 1'-acetoxychavicol acetate, polygodial, warburganal, muzigadial, merulidial, acetylmerulidial, aframodial, cinnamodial, cinnamolide, cinnamosmolide, capsicodendrin, velleral, isovelleral, isoisovelleral, isodrimeninol,

echinacea extract, jambu oleoresin, ginger oleoresin, ginger extract, pepper extract (Piper spp., in particular P. nigrum, P. hispidum, P. tuberculatum, P. longum, P. arboreum, P. futokadsura, P. guineense, P. sarmentosum), black pepper extract, red pepper oleoresin, red pepper oil, Japanese pepper extract, extracts of toothache grass (Ctenium aromaticum), extracts of tarragon (Artemisia dracunculus), pellitory root extracts (Anacyclus spp., in particular Anacylcus pyrethrum L.), echinacea extracts (Echinaceae spp., for example E. angustifolia), extracts of Szechuan pepper (Zanthoxylum spp., in particular Zanthoxylum piperitum, Z. clava-herculin, Z. bungeanum, Z. zanthoxyloides), Spilanthes or paracress extracts (Spilanthes spp., in particular Spilanthes acmella), extracts of Acmella spp. (for example A. ciliata), extracts of Achillea spp. (for example Achillea wilsoniana), extracts of Fagara species (Fagara zanthoxyloides), extracts of Heliopsis spp. (preferably H. longipes), extracts of Cissampelos glaberrima, extracts of Dinosperma erythrococca, bark extracts of Esenbeckia alata, extracts of Stauranthus perforatus, extracts of water pepper (Persicaria hydropiper),
and/or

(B-2) comprises or consists of methanethiol (methyl mercaptan), ethanthiol (ethyl mercaptan), propanethiol, isopropylthiol, allyl mercaptan, furfuryl mercaptan, 5-methyl-2-furfurylthiol, dimethyl sulfide, diallyl sulfide, methyl propyl sulfide, methyl isopropyl sulfide, dimethyl disulfide, diethyl sulfide, dipropyl disulfide, diallyl disulfide, allyl propyl disulfide, methyl isopropyl disulfide, methyl propyl disulfide, propyl propenyl disulfide, difurfuryl disulfide, furfuryl methyl disulfide (methyl 2-furyl methyl disulfide), bis(2-methyl-3-furyl) disulfide, dimethyl trisulfide, diisopropyl trisulfide, dipropyl trisulfide, allyl methyl trisulfide, ethyl methyl trisulfide (2,3,4-trithiahexane), diallyl trisulfide, 2,3,5-trithiahexane (methyldisulfanyl-methylsulfanylmethane), allyl isothiocyanate, phenylethyl isothiocyanate, cyclopropyl isothiocyanate, butyl isothiocyanate, 3-methylthiopropyl isothiocyanate, 4-hydroxybenzyl isothiocyanate, 4-methoxybenzyl isothiocyanate, sulforaphane (1-isothiocyanato-4-methylsulfinylbutane), 3-methylthiopropanal (3-methylsulfanyl propanal; methional), 3-(methylthio) hexan-1-ol, 3-mercaptohexan-1-ol, 3-mercaptohexyl acetate, 3-mercaptohexyl butyrate, S-methyl thioacetate, 2-methylthiophene, 2-ethylthiophene, 2-propylthiophene, 2-butylthiophene, 2-hexylthiophene, 2-octylthiophene, 2,5-dimethylthiophene, 2-acetylthiophene, 3-(methylthiomethyl)thiophene, 2-thiophenyl methanethiol, 3-methyl-2-thiophenyl methanethiol, methyl-1,3-dithiolane, 3,5-dimethyl-1,2,4-trithiolane, allicin (2-propene-1-sulfinothioic acid S-2-propenyl ester), ajoene ((E/Z)-4,5,9-trithiadodeca-1,6,11-triene 9-oxide), 3-[(prop-2-ene-1-sulfinyl)sulfanyl]prop-1-ene, 3-vinyl-(4*H*)-1,2-dithiin, 2-vinyl-(4*H*)-1,3-dithiin, syn-propanethial S-oxide, syn-butanethial S-oxide, 2,4,6-trimethyl-perhydro-1,3,5-dithiazine, 2,4,6-trimethyldihydro-4H-1,3,5-dithiazine, 2,4,6-triethyldihydro-4H-1,3,5-dithiazine, 2-isopropyl-4,6-dimethyldihydro-1,3,5-dithiazine, 4-isopropyl-2,6-dimethyldihydro-1,3,5-dithiazine, 2,4,6-triisobutyl-5,6-dihydro-4H-1,3,5-dithiazine, 2-methyl-(3-methylthio)-furan, 2-methylfuran-3-thiol, 2-methyl-3-methylthiofuran, 2-methyltetrahydrofuran-3-thiol, 2,5-dimethylfuran-3-thiol, 2-methyl-3-(methyldisulfanyl)furan, 2-methyl-3-(methyl dithio)furan, 1,4-dithiane, 2,5-dihydroxy-1,4-dithiane, and 2,5-dihydroxy-2,5-dimethyl-1,4-dithiane,
and/or

(B-3) comprises or consists of aroma molecules selected from the group consisting of 2-acetyl-1-pyrroline, 2-propionylpyrroline, 2-acetylpyrrole, pyridine, 2-methylpyridine, 3-methylpyridine, 6-acetyl-2,3,4,5-tetrahydropyridine, 2-pentylpyridine, 2-(3-phenylpropyl)pyridine, furfural, 5-hydroxymethylfurfural, 2,5-dimethylfuran, 2-methyl-5-ethylfuran, 2-ethylfuran, 2-propylfuran, 2-butylfuran, 2-pentylfuran, 2-pentenylfuran, 2-acetylfuran, 4-hydroxy-5-methyl-3(2*H*)-furanone, 2,5-dimethyl-4-hydroxy-(2*H*)furan-3-one, 4,5-dimethyl-3-hydroxy-2(5*H*)furanone, 2,4-dimethyl-3-oxazoline, 2,4-dimethyl-5-ethyl-3-oxazoline, 2,5-dimethyl-4-ethyl-3-oxazoline, 2,4,5-trimethyl-3-oxazoline, 2-acetyl-2-thiazoline, 2-propionyl-2-thiazoline, 2,4-dimethyl-3-thiazoline, 2,4,5-trimethylthiazoline, 2-isobutyl-4,5-dimethyl-3-thiazoline, 4-methylthiazole, 2-methylthiazole, 2-acetyl-2-thiazole, 2,4,5-trimethylthiazole, 2,4-dimethyl-5-ethylthiazole, 5-hydroxyethyl-4-methylthiazole (sulfurol), 1,3-benzothiazole, 1,3-thiazolidine, 2-methyl-1,3- thiazolidine, 2-isopropyl-1,3-thiazolidine,

2-methylpyrazine, 2-ethylpyrazine, 2-propylpyrazine, 2-isopropylpyrazine, 2-methoxypyrazine, 2,3-dimethylpyrazine, 2,5-dimethylpyrazine, 2,6-dimethylpyrazine, 2,3,5-trimethylpyrazine, 2,3,5,6-tetramethylpyrazine, 2-methyl-3-ethylpyrazine 2-ethyl-3,5-dimethylpyrazine, 2-ethyl-3,6-dimethylpyrazine, 3-ethyl-2,5-dimethylpyrazine, 2,3-diethyl-5-methylpyrazine, 3-isobutyl-2-methoxypyrazine, 3-isopropyl-2-methoxypyrazine, 2-acetylpyrazine, 2-acetyl-3-methylpyrazine, 5-acetyl-2-methylpyrazine, 6-acetyl-2-ethylpyrazine,

2-methylpropanal, 2-methybutanal, 3-methylbutanal, 4-pentenal, (E)-2-nonenal, hexanal, (E)-2-hexenal, 2,4-hexadienal, heptanal, 4-heptenal, octanal, (E)-2-octenal, octadienal, nonanal, (E,E)-2,4-nonadienal, (2E,6Z)-nonadienal, (E,E)-2,4-decadienal, (E,Z)-2,4-decadienal, (E)-2-undecenal, 12-methyl-tridecanal,

3-methyl-1-butanol, 1-pentanol, 1-penten-3-ol, 3-cis-hexenol, 3-methyl-2-hexen-1-ol, 1-heptanol, 3-octanol, 1-octene-3-ol,

3-hydroxy-2-butanone (acetoin), 2-butanone, 2,3-butanedione (diacetyl), 2-pentanone, methyl isobutyl ketone, 1-pentene-3-one, 2-hexanone, oct-1-en-3-one, 2,3-octanedione, 2-nonanone, 2-decanone, 4-undecanone, 2-tridecanone, 3-methylcyclopentanone, 3-methylcyclopentenone, methyl cyclopentenolone (3-methyl-1,2-cyclopentanedione), dimethyl cyclopentenolone (3,4-dimethyl-1,2-cyclopentanedione, 3,5-dimethyl-1,2-cyclopentanedione), ethyl cyclopentenolone (3-ethyl-1,2-cyclopentanedione),

gamma-hexalactone, gamma-octalactone, gamma-decalactone, gamma-dodecalactone, delta-nonalactone,

butyric acid, isobutyric acid, isovaleric acid, pentanoic acid, hexanoic acid, isocaproic acid, heptanoic acid,

4-ethyl-1-methylhexane, 1,1,3-trimethylcyclohexane, naphthalene, alpha-pinene, beta-pinene, 3,6-dimethylundecane,

palmitic acid, stearic acid, linoleic acid, vaccenic acid, 11,14-eicosdienoic acid, arachidonic acid, 5,8,11,14,17-eicosapentaenoic acid,

phenol, o-cresol, m-cresol, p-cresol, guaiacol, 4-methylguaiacol, 4-ethylguaiacol, 4-propylguaiacol, 4-vinylguaiacol, 1,2-dihydroxybenzene, vanillin, eugenol, (E)-isoeugenol, (Z)-isoeugenol, acetovanillone, 4-(2-propio)-vanillone, 4-(1-propio)-vanillone, 2,6-dimethoxyphenol (syringol), 4-methylsyringol, 4-ethylsyringol, 4-propylsyringol, syringaldehyde, 4-acetosyringol, 4-(2-propio)-syringone, 4-(1-propio)-syringone, 4-(2-propenyl)-syringol, (Z)-4-(1-propenyl)-syringol, (E)-4-(1-propenyl)-syringol, and 4-acetosyringone,
and/or

(B-4) comprises or consists of aroma molecules selected from the group consisting of

eugenol, cinnamaldehyde (preferably trans-cinnamaldehyde), anisole, anethole (preferably trans-anethole), estragole, thymol, carvacrol, vanillin, maltol, ethyl maltol, nerol, citral, citronellal, citronellol, nerolidol, linalool, massoia lactone, sotolon, caryophyllene, alpha-phellandrene, terpinene-4-ol, and gamma-nonalactone,
and/or

(B-5) comprises or consists of amino acids selected from the group consisting of arginine, alanine, valine, isoleucine, leucine, methionine, phenylalanine, tyrosine, tryptophan, serine, asparagine, aspartic acid, threonine, cysteine, proline, 4-hydroxyproline, glutamine, lysine, 5-hydroxylysine, histidine, anserine, carnosine, S-allylmercaptocysteine, S-allylcysteine, alliin, taurine, gamma-amino acids (preferably gamma-amino butyric acid) and the physiologically acceptable salts thereof, preferably the hydrochlorides thereof,
and/or

(B-6) comprises or consists of the group consisting of sodium lactate, potassium lactate, sodium chloride, potassium chloride, hexametaphosphate salts, sodium and/or potassium salts of tripolyphosphate, tetrasodium pyrophosphate, monosodium glutamate, inosinic acid (inosine monophosphate), disodium inosinate, guanosine monophosphate, and disodium guanylate,
and/or

(B-7) substances selected from the group consisting of beef extract, soy sauce, soy sauce extract, soy protein, yeast extract, autolyzed yeast extract, malt extract, hydrolyzed vegetable protein, whey protein, preferably in dry, more preferably in powdered form.

A more preferred flavoring mixture according to the present invention comprises one or more, preferably two or more, more preferably three or more additional ingredients selected from the group consisting of constituents (B-1), (B-2), (B-3), (B-4), and (B-5), wherein constituent

(B-1) comprises or consists of substances selected from the group consisting of spilanthol (2E,6Z,8E-decatrienoic acid N-isobutylamide), 2E,6Z,8E,10E-dodecatetraenoic acid N-(2-methylpropyl)amide ([alpha]-sanshool), 2E,6Z,8E,10E-dodecatetraenoic acid N-(2-hydroxy-2-methylpropyl)amide [alpha]-hydroxysanshool), 2E,6E,8E,10E-dodecatetraenoic acid N-(2-hydroxy-2-methylpropyl)amide ([gamma]-hydroxysanshool), 2E,4E,8Z,10E,12E-tetradecapentaenoic acid N-(2-hydroxy-2-methylpropyl)amide ([gamma]-hydroxysanshool), 2E,4E,8Z,10E,12E-tetradecapentaenoic acid N-(2-methylpropyl)amide ([gamma]-sanshool), 2E,4E,8Z,11Z-tetradecatetraenoic acid N-(2-hydroxy-2-methylpropyl)amide (bungeanool), 2E,4E-decadienoic acid N-isobutylamide (trans-pellitorine), 2E,4E-decadienoic acid N-piperide (sarmentine),

6-gingerol, 8-gingerol, 10-gingerol, gingerdione-[2], gingerdione-[3], gingerdione-[4], gingerdione-[6], gingerdione-[8], gingerdione-[10], gingerdione-[12], [6]-shogaol, [8]-shogaol, [10]-shogaol, [6]-paradol, [8]-paradol, [10]-paradol, zingerone, capsaicin, dihydrocapsaicin, nordihydrocapsaicin, homocapsaicin, homodihydrocapsaicin,
chavicine, piperine,
and/or

(B-2) comprises or consists of methanethiol (methyl mercaptan), ethanthiol (ethyl mercaptan), propanethiol, isopropylthiol, allyl mercaptan, furfuryl mercaptan, 5-methyl-2-furfurylthiol, dimethyl sulfide, diallyl sulfide, methyl propyl sulfide, methyl isopropyl sulfide, dimethyl disulfide, diethyl sulfide, dipropyl disulfide, diallyl disulfide, allyl propyl disulfide, methyl isopropyl disulfide, methyl propyl disulfide, propyl propenyl disulfide, difurfuryl disulfide, furfuryl methyl disulfide (methyl 2-furyl methyl disulfide), bis(2-methyl-3-furyl) disulfide, dimethyl trisulfide, diisopropyl trisulfide, dipropyl trisulfide, allyl methyl trisulfide, diallyl trisulfide, 2,3,5-trithiahexane (methyldisulfanyl-methylsulfanylmethane), allyl isothiocyanate, 3-methylthiopropyl isothiocyanate, 3-methylthiopropanal (3-methylsulfanyl propanal; methional), 3-(methylthio) hexan-1-ol, 3-mercaptohexan-1-ol, 3-mercaptohexyl acetate, 2-acetylthiophene, 3-(methylthiomethyl)thiophene, allicin (2-propene-1-sulfinothioic acid S-2-propenyl ester), 2-methyl-(3-methylthio)-furan, 2-methylfuran-3-thiol, 2-methyl-3-methylthiofuran, 2-methyltetrahydrofuran-3-thiol, 2,5-dimethylfuran-3-thiol, 2-methyl-3-(methyldisulfanyl)furan, 2-methyl-3-(methyl dithio)furan, 1,4-dithiane, 2,5-dihydroxy-1,4-dithiane, and 2,5-dihydroxy-2,5-dimethyl-1,4-dithiane,
and/or

(B-3) comprises or consists of aroma molecules selected from the group consisting of 2-acetyl-1-pyrroline, 2-propionylpyrroline, 2-acetylpyrrole, pyridine, 2-methylpyridine, 3-methylpyridine, 6-acetyl-2,3,4,5-tetrahydropyridine, 2-pentylpyridine, 2-(3-phenylpropyl)pyridine, 5-hydroxymethylfurfural, 2-pentylfuran, 2-pentenylfuran, 2-acetylfuran, 4-hydroxy-5-methyl-3(2*H*)-furanone, 2,5-dimethyl-4-hydroxy-(2*H*)furan-3-one, 4,5-dimethyl-3-hydroxy-2(5*H*)furanone, 2,4-dimethyl-3-oxazoline, 2,4-dimethyl-5-ethyl-3-oxazoline, 2,5-dimethyl-4-ethyl-3-oxazoline, 2,4,5-trimethyl-3-oxazoline, 2-acetyl-2-thiazoline, 2-propionyl-2-thiazoline, 2,4-dimethyl-3-thiazoline, 2-isobutyl-4,5-dimethyl-3-thiazoline, 4-methylthiazole, 2-methylthiazole, 2-acetyl-2-thiazole, 2,4,5-trimethylthiazole, 2,4-dimethyl-5-ethylthiazole, 5-hydroxyethyl-4-methylthiazole (sulfurol),

3-isobutyl-2-methoxypyrazine, 3-isopropyl-2-methoxypyrazine, 2-acetylpyrazine, 2-acetyl-3-methylpyrazine, 5-acetyl-2-methylpyrazine, 6-acetyl-2-ethylpyrazine,

(E,E)-2,4-nonadienal, (2E,6Z)-nonadienal, (E,E)-2,4-decadienal, (E,Z)-2,4-decadienal, (E)-2-undecenal, 12-methyl-tridecanal,

3-hydroxy-2-butanone (acetoin), 2-butanone, 2-pentanone, 1-pentene-3-one, oct-1-en-3-one, 2,3-octanedione, 2-nonanone, 2-decanone, 4-undecanone, 2-tridecanone,

gamma-decalactone, gamma-dodecalactone, delta-nonalactone,

isovaleric acid, pentanoic acid, hexanoic acid, isocaproic acid, heptanoic acid,

3,6-dimethylundecane,

4-methylguaiacol, 4-ethylguaiacol, 4-propylguaiacol, 4-vinylguaiacol, 1,2-dihydroxybenzene, (E)-isoeugenol, (Z)-isoeugenol, acetovanillone, 2,6-dimethoxyphenol (syringol), syringaldehyde, 4-acetosyringol, and 4-acetosyringone,
and/or

(B-4) comprises or consists of aroma molecules selected from the group consisting of

eugenol, cinnamaldehyde (preferably trans-cinnamaldehyde), anisole, anethole (preferably trans-anethole), thymol, vanillin, maltol, ethyl maltol, and sotolon,
and/or

(B-5) comprises or consists of amino acids selected from the group consisting of alanine, valine, isoleucine, leucine, methionine, phenylalanine, tyrosine, tryptophan, threonine, cysteine, proline, 4-hydroxyproline, lysine, 5-hydroxylysine, carnosine, S-allylmercaptocysteine, S-allylcysteine, alliin, taurine, gamma-amino butyric acid, and the physiologically acceptable salts thereof, preferably the hydrochlorides thereof.

An even more preferred flavoring mixture according to the present invention comprises one or more, preferably two or more, more preferably three or more additional ingredients selected from the group of constituents (B-1) to (B-7) consisting of spilanthol, 6-gingerol, 8-gingerol, gingerdione-[6], gingerdione-[8], [6]-shogaol, [8]-shogaol, [6]-paradol, [8]-paradol, zingerone, capsaicin, dihydrocapsaicin, nordihydrocapsaicin, chavicine, piperine, allyl mercaptan, furfuryl mercaptan, 5-methyl-2-furfurylthiol, diallyl sulfide, dipropyl disulfide, diallyl disulfide, difurfuryl disulfide, dimethyl trisulfide, diisopropyl trisulfide, dipropyl trisulfide, allyl methyl trisulfide, diallyl trisulfide, allyl isothiocyanate, 3-methylthiopropyl isothiocyanate, 3-methylthiopropanal (3-methylsulfanyl propanal; methional), 2-acetyl-1-pyrroline, 2-pentylfuran, 2-acetylfuran, 4-hydroxy-5-methyl-3(2*H*)-furanone, 2,5-dimethyl-4-hydroxy-(2*H*)furan-3-one, 4,5-dimethyl-3-hydroxy-2(5H)furanone, 2,4-dimethyl-3-oxazoline, 2,4,5-trimethyl-3-oxazoline, 2-acetyl-2-thiazoline, 2-isobutyl-4,5-dimethyl-3-thiazoline, 5-hydroxyethyl-4-methylthiazole (sulfurol), 3-isobutyl-2-methoxypyrazine, 3-isopropyl-2-methoxypyrazine, 2-acetylpyrazine, (2E,6Z)-nonadienal, (E,E)-2,4-decadienal, (E,Z)-2,4-decadienal, 12-methyl-tridecanal, 2-nonanone, 2-decanone, 4-undecanone, 2-tridecanone, hexanoic acid, isocaproic acid, heptanoic acid, 4-methylguaiacol, 4-vinylguaiacol, (E)-isoeugenol, acetovanillone, 2,6-dimethoxyphenol (syringol), 4-acetosyringol, 4-acetosyringone, eugenol, cinnamaldehyde, anisole, sotolon, vanillin, linalool, methionine, cysteine, proline, 4-hydroxyproline, lysine, 5-hydroxylysine, carnosine, S-allylmercaptocysteine, S-allylcysteine, alliin, taurine, gamma-amino butyric acid, beef extract, soy sauce, powdered soy sauce, and yeast extract.

If a flavoring mixture according to the present invention contains one or more additional ingredients selected from the group of capsaicin, dihydrocapsaicin, nordihydrocapsaicin, chavicine, piperine, and allyl isothiocyanate, the total amount of the said additional ingredients preferably is 1 ppm or more, more preferably 10 ppm or more, more preferably 25 ppm or more, even more preferably 50 ppm or more, particularly preferably 100 ppm or more, in each case based on the total weight of the flavoring mixture.

A preferred flavoring mixture according to the present invention comprises three, four, five, six, seven, eight, nine, ten or more additional ingredients selected from the group consisting of constituents (B-1), (B-2), (B-3), (B-4), (B-5), (B-6), and (B-7), preferably selected from the preferred or particularly preferred constituents indicated above.

A preferred flavoring mixture according to the present invention comprises (i) one or more, preferably two or more, more preferably three or more additional ingredients selected from the group consisting of constituents (B-1), (B-2), (B-3), (B-4), (B-5), (B-6), and (B-7), and (ii) one or more, preferably two or more additional ingredients selected from the group consisting of constituents (B-8) and (B-9):

(B-8) solid carrier substances selected from the group consisting of chemically modified starches, degraded starches, pectins, glutens, starch hydrolysates, chemically modified celluloses, gums, proteins, silicon dioxide (silica, silica gel), cyclodextrins, and milk powders,
and/or

(B-9) food acids, preferably selected from the group consisting of acetic acid, adipic acid, caffeotannic acid, citric acid, iso-citric acid, fumaric acid, galacturonic acid, glucuronic acid, glyceric acid, glycolic acid, lactic acid, malic acid, oxalic acid, pyruvic acid, quinic acid, shikimic acid, succinic acid, tannic acid, ascorbic acid and tartaric acid, and the physiologically acceptable salts thereof.

In a preferred embodiment, constituent (B-8) is selected from the group consisting of chemically modified starches (preferably chemically modified corn starch), dextrins, maltodextrins (preferably obtained from corn, tapioca, cassava, rice, sago, potato, sweet potato, wheat, buckwheat, rye, oat, yam, chestnut, water chestnut, peas or beans (such as mung beans)), hydrolyzed corn gluten, gum Arabic (acacia gum), gum traganth, gum ghatti, gum karaya, gellan gum, xanthan gum, guar gum, agar-agar, alginates, gelatins, carrageenan, beta-cyclodextrin, gamma-cyclodextrin, silica, methylcellulose, hydroxypropyl cellulose, hydroxypropyl methylcellulose, carboxymethyl cellulose, sodium carboxymethyl cellulose, milk powder, and nonfat dry milk.

Constituent (B-8) is preferably selected from the group consisting of are silicon dioxide, gum Arabic and maltodextrins, maltodextrins having DE (Dextrose Equivalent) values in the range of from 5 to 22 in turn being preferred, maltodextrins having DE values in the range of from 10 to 20 being even more preferred. It is in this case not critical which plant originally provided the starch for producing the starch hydrolysates. Suitable and readily available are corn-based starches and starches made up of tapioca, rice, wheat or potatoes.Constituent (B-8) may simultaneously act as anticaking agent (flow auxiliary, for example in case silicon dioxide is used.

In a preferred embodiment, the one or more food acids from constituent (B-9) are selected from the group consisting of citric acid, lactic acid, malic acid, succinic acid, fumaric acid, tartaric acid, ascorbic acid or adipic acid, and the physiologically acceptable salts thereof.

A flavoring mixture according to the present invention preferably imparts one or more sensory impressions selected from the group of meat, seafood, fish, seaweed, mushrooms and *Allium* species, preferably selected from the group consisting of (respectively raw, cooked, fried, baked, roasted and/or smoked) onion, garlic, leek, shallot, beef, pork, chicken, turkey, duck, goat, lamb, mutton, deer, springbok, catfish, tuna, cod, eel, sword fish, hake, salmon, trout, tilapia, whiting, mussel, scallop, clam shell, shrimp, prawn, squid, crab, button mushroom, brown mushroom, black ear mushroom, shiitake mushroom, chanterelle mushroom, chestnut mushroom, clamshell mushroom, cloud ear mushroom (mo-er mushroom), enoki mushromms, eryngii mushroom, gamboni mushroom, maitake mushroom, matsutake mushroom, morels, nameko mushroom, oyster mushroom, porcini mushroom, portobello mushroom, puff ball mushroom, and straw mushroom.

In a further aspect, the present invention relates to a composition for oral consumption, comprising
(a) a sensorially effective amount of
   - a compound of Formula (I) or of a mixture of compounds of Formula (I) as defined hereinbefore,
      or
   - a flavoring mixture according to the present invention as defined above,
      and
(b) one or more further constituents suitable for oral consumption.

The compositions according to the invention, which are suitable for oral consumption and used for nutrition, oral care, or consumption for pleasure, are in many cases products which are intended to be introduced into the human oral cavity, to remain there for a specific time and subsequently to be either eaten (for example ready-to-eat food products, see also below) or removed again from the oral cavity (for example chewing gums or toothpaste). These products include in this case all substances or goods which are intended for human consumption in a processed, partly processed or unprocessed state. This also includes substances which are added to food products during the production, processing or treatment thereof and are intended to be introduced into the human oral cavity.

Within the scope of the present text, the terms "for oral consumption", "for oral use", "food product" and the like refer in particular to substances or mixtures which are intended to be swallowed by a human being in an unchanged, prepared or processed state and then to be digested. Thus, these terms also refer to casings, coatings or other encapsulations which are intended also to be swallowed, or in which swallowing is likely. Specific products which are conventionally removed from the oral cavity again (for example chewing gums) are also to be regarded as being "for oral consumption", "for oral use", or a "food product" within the scope of the present text, as there is a possibility that they are at least partly swallowed.

The term a "ready-to-eat food product" refers in this case to a product, the composition of which, in terms of the substances which determine the flavor, is already complete. The term "ready-to-eat food product" also includes beverages and solid or semisolid ready-to-eat food products. Examples include deep-frozen products which, prior to consumption, have to be defrosted and heated to eating temperature. The ready-to-eat food products also include products such as yoghurt or ice cream but also chewing gums or hard caramels.

The term "semifinished product" refers in connection with the present text to a product which on account of its very high content of flavoring substances is unsuitable for use as a ready-to-eat food product. It is only by mixing with at least one other constituent (i.e by reducing the concentration of the respective aroma and flavoring substances) and, if appropriate, further process steps (e.g heating, freezing) that the semifinished product is converted to a ready-to-eat food product. Examples of semifinished products are packet soups, baking flavorings and custard powder.

The term an "oral care product" (also referred to as an oral hygiene product) refers in the sense of the invention to one of the formulations with which a person skilled in the art is familiar for cleaning and caring for the oral cavity and the pharynx and for freshening breath. This expressly includes care for teeth and gums. Conventional oral hygiene products are administered in particular in the form of creams, gels, pastes, foams, emulsions, suspensions, aerosols, sprays, and also capsules, granules, lozenges, tablets, sweets or chewing gums, although this list should not be understood to limit this invention.

Preferred oral care products (oral hygiene product) include in particular those in the form of toothpaste, dental cream, dental gel, dental powder, tooth-cleaning liquid, tooth-cleaning foam, mouthwash, dental cream and mouthwash as a 2-in-1 product, oral spray, or dental care chewing gum.

Chewing gums generally comprise a chewing gum base, i.e. a masticatory substance which becomes plastic during chewing, a sweet tasting compound or mixture of compounds, like sugars, sugar substitutes, and/or sugar alcohols (in particular sorbitol, xylitol, mannitol), one or more cooling agents, optionally warming agents, humectants, thickeners, emulsifiers, decaking agents, and one or more flavorings (for example: eucalyptus-menthol, cherry, strawberry, grapefruit, vanilla, banana, citrus, peach, blackcurrant, tropical fruits, ginger, coffee, clove, cinnamon).

A large number of different chewing gum bases are known in the art. Today, conventional chewing gum bases usually comprise, in addition to traditionally used natural resins or the natural latex chicle, elastomers such as polyvinyl acetates, polyethylenes, (low or medium molecular weight) polyisobutenes, polybutadienes, isobutene/isoprene copolymers (butyl rubber), polyvinyl ethyl ethers, polyvinyl butyl ethers, copolymers of vinyl esters and vinyl ethers, styrene/butadiene copolymers (styrene/butadiene rubbers) or vinyl elastomers, for example based on vinyl acetate/vinyl laurate, vinyl acetate/vinyl stearate or ethylene/vinyl acetate, and mixtures of the aforementioned elastomers, as described for example in EP 0 242 325, US 4,518,615, US 5,093,136, US 5,266,336 US 5,601,858 or US 6,986,709. In addition, chewing gum bases comprise further constituents such as for example (mineral) fillers, plasticizers, emulsifiers, antioxidants, waxes, fats or fatty oils, such as for example hardened (hydrogenated) vegetable or animal fats, monoglycerides, diglycerides or triglycerides. Suitable (mineral) fillers include for example calcium carbonate, titanium dioxide, silicon dioxide, talc, aluminum oxide, dicalcium phosphate, tricalcium phosphate, magnesium hydroxide and mixtures thereof. Suitable plasticizers or agents for preventing sticking (detackifiers) include for example lanolin, stearic acid, sodium stearate, ethyl acetate, diacetin (gylcerol diacetate), triacetin (gylcerol triacetate), triethyl citrate. Suitable waxes include for example paraffin waxes, candelilla wax, carnauba wax, microcrystalline waxes and polyethylene waxes. Suitable emulsifiers include for example phosphatides such as lecithin, monoglycerides and diglycerides of fatty acids, for example glycerol monostearate.

A number of compositions according to the invention are preferred. Particularly preferred is a (preferably spray-dried) composition comprising, in addition to a sensorially effective amount of one or more compounds of Formula (I) or a mixture thereof, and one or more solid excipients suitable for consumption. Preferred compositions consist of the compound or compounds of Formula (I) in accordance with the invention and the excipient or excipients. In this case, that which was stated hereinbefore applies accordingly with regard to compounds of Formula (I) and preferred mixtures.

A flavoring mixture according to the present invention or a composition for oral consumption according to the present invention (each as defined above) at 25°C and 1013 mbar preferably are solid, preferably in powdered form, preferably in spray-dried form. With regard to spray drying, reference may be made to US 3,159,585, US 3,971,852, US 4,532,145 or US 5,124,162.

A flavoring mixture according to the present invention or a composition for oral consumption according to the present invention preferably comprises a total amount of the compounds of Formula (I) as defined above in the range of from 250 ppm to 25 wt.%, preferably of from 500 ppm to 15 wt.%, more preferably of from 1000 ppm to 10 wt.%, particularly preferably of from 2000 ppm (corresponding to 0.20 wt.%) to 5 wt.%.

Examples of advantageous excipients in preferred (preferably spray-dried) compositions according to the invention include silicon dioxide (silica, silica gel), carbohydrates and/or carbohydrate polymers (polysaccharides), cyclodextrins, starches, degraded starches (starch hydrolysates), chemically or physically modified starches, modified celluloses, gum Arabic, gum ghatti, gum tragacanth, gum karaya, carrageenan, guar flour, carob flour, alginates, pectin, inulin or xanthan gum. Preferred starch hydrolysates include maltodextrins and dextrins.

Preferred excipients are silicon dioxide, gum Arabic and maltodextrins, maltodextrins having DE values in the range of from 5 to 22 being preferred in this case too. It is in this case immaterial which plant originally provided the starch for producing the starch hydrolysates. Suitable and readily available are corn-based starches and starches made up of tapioca, rice, wheat or potatoes. The excipients can in this case also act as a flow auxiliary, such as for example silicon dioxide. The compositions according to the invention, which also comprise, in addition to the compound or compounds of Formula (I) in accordance with the invention, one or more solid excipients, can be produced for example by mechanical mixing processes, wherein the present particles can at the same time also be size-reduced, or by means of spray-drying.

Compositions according to the invention which comprise solid excipients and are produced by means of spray drying are preferred.

Preferred compositions according to the invention which comprise excipients and are produced by means of spray drying have an average particle size in the range of from 30 to 300 µm and residual moisture of less than or equal to 5% by weight.

The ratio by weight of the total mass of the compound of Formula (I) or - if mixtures of compounds of formula (I) are used - the ratio by weight of the total mass of the compounds of Formula (I) to the solid excipient or excipients suitable for consumption lies preferably in the range of from 1 : 10 to 1 : 50000, more preferably in the range of from 1 : 50 to 1 : 20000, even more preferably in the range of from 1 : 100 to 1 : 10000, particularly preferably in the range of from 1 : 500 to 1 : 5000, based on the dry (i.e. essentially water-free, preferably water-free) mass of the composition.

In certain embodiments, in a composition according to the invention, the sum of the constituents comprising (i) compounds of Formula (I) and (ii) excipients lies preferably in the range of from 70 to 100% by weight, preferably in the range of from 85 to 100% by weight.

In preferred embodiments, a composition according to the present invention comprises one or more further constituents suitable for consumption, preferably
- one or more emulsifiers, preferably selected from the group consisting of lecithins (preferably naturally occurring lecithins, particularly lecithin from egg or soy), phospholipids (preferably phosphatidylcholines), monoacylglycerols, and diacylglycerols,
   and/or
- one or more vegetable fatty oils, preferably selected from the group consisting of soy oil (soybean oil), olive oil, sunflower oil, safflower oil, corn oil, coconut oil, peanut oil, hazelnut oil, almond oil, walnut oil, palm kernel oil, pumpkin seed oil, canola oil (rapeseed oil), sesame oil, cottonseed oil, wheat germ oil, linseed oil, and rice bran oil,
   and/or
- one or more preservatives, preferably selected from the group consisting of benzoic acid, sorbic acid, phosphoric acid, and the physiologically acceptable salts of these acids, preferably sodium benzoate, potassium benzoate, sodium sorbate, potassium sorbate, monosodium phosphate, disodium phosphate, trisodium phosphate, monopotassium phosphate, dipotassium phosphate, and/or tripotassium phosphate),
   and/or
- one or more antioxidants and optionally one or more substances for intensifying the antioxidative effect of said antioxidants,
   and/or
- one or more vitamins and the physiologically acceptable salts or esters thereof, preferably selected from the group consisting of vitamin A, vitamin A palmitate, vitamin B1, vitamin B2 (riboflavin), vitamin B3 (niacin), vitamin B6, vitamin B9 (folic acid) vitamin B12, vitamin C, monosodium ascorbate, monopotassium ascorbate, calcium diascorbate, magnesium diascorbate, ascorbyl palmitate, ascorbyl stearate, vitamin D, and vitamin E, vitamin E acetate, vitamin E palmitate, vitamin H (biotin),
   and/or
- one or more essential oils, preferably selected from the group consisting of aniseed oil, basil oil, bergamot oil, curry leaf oil, winter savory oil, summer savory oil, bucco-leaf oil, cardamom oil, lemon oil, coriander oil, costus root oil, cumin oil, dill oil, dillseed oil, tarragon oil, fennel oil, asafoetida oil, ginger oil, garlic oil, onion oil, leek oil, shallot oil, carrot-seed oil, caraway oil, lemongrass oil, distilled lime oil, pressed lime oil, bay-leaf oil, mace oil, marjoram oil, nutmeg oil, myrtle oil, clove leaf oil, clove flower oil, neroli oil, orange oil, origanum oil, parsley leaf oil, parsley seed oil, pepper oil, pimento oil, pine oil, rosemary oil, celery seed oil, tagetes oil, tea-tree oil, thyme oil, verbena oil, juniper oil, hyssop oil, cinnamon leaf oil, cinnamon bark oil,
   and/or
- one or more coloring agents, preferably selected form the group consisting of carotenes (E-number E160a, preferably beta-carotene), paprika extract (E-number E160c), red beet juice powder (comprising betanine, beetroot red, E-number E162), annatto (E-number E160b), anthocyanins (E-number E163), chlorophylls (E-number E140), turmeric (E-number E100, comprising curcumin), tartrazine (E-number E102), amaranth (E-number E123), titanium dioxide (E-number E171), iron oxides and iron hydroxides (E-number E172), erythrosine (E-number E127), and caramel (E-number E150).

It is also possible and in some cases preferred to combine or (wholly or partially) replace the one or more vegetable fatty oils optionally used in the context of the present invention with liquid neutral esters based on medium-chain fatty acids and glycerol, such as for example Miglyols (for example Miglyol 810, Miglyol 812). Use is preferably also made of fractionated coconut oils having mainly fatty acid radicals containing 6 to 8 carbon atoms. These are distinguished by their flavor neutrality and by their good oxidation stability.

In another aspect, the present invention relates to a ready-to-eat product, a ready-to-use product, or a semifinished product for oral use or oral consumption, comprising a sensorially effective amount of
- a compound of Formula (I) or a mixture of compounds of Formula (I), or
- a flavoring mixture according to the present invention as defined above, preferably in one of the preferred or particularly preferred embodiments, or
- a composition for oral consumption according to the present invention as defined above, preferably in one of the preferred or particularly preferred embodiments,
   and one or more further ingredients for oral consumption selected from the group consisting of base or bulk materials, additives, excipients, and functional ingredients.

A further preferred composition according to the invention, which is suitable for consumption and comprises (a) a compound of Formula (I) in accordance with the invention or (b) a mixture of compounds of Formula (I), is a water-in-oil (W/O) emulsion. In addition to the compound of Formula (I) or to the above-defined mixtures, an emulsion of this type comprises water, an oil phase, one or more W/O emulsifiers, optionally one or more antioxidants and optionally one or more substances for intensifying an antioxidative effect.

Preferably, a composition according to the invention of this type (W/O emulsion) comprises, based on the total weight of the composition,
- a total of from 0.01 to 0.1% by weight of compounds of Formula (I),
- 5 to 30% by weight, preferably 8 to 25% by weight of water,
- 50 to 90% by weight, preferably 60 to 80% by weight of an oil phase,
- 0.1 to 5% by weight of an edible W/O emulsifier,
and optionally one or more antioxidants and optionally one or more substances for intensifying an antioxidative effect.

In a preferred embodiment, a W/O emulsion according to the invention of this type consists of the aforementioned constituents in the aforementioned amounts.

Preferably, the edible W/O emulsifier is selected from the group consisting of lecithin (E 322), monoglycerides and diglycerides of edible fatty acids (E 471), acetic acid monoglycerides (E 472a), lactic acid monoglycerides (E 472b), citric acid monoglycerides (E 472c), tartaric acid monoglycerides (E 472d), diacetyl tartaric acid monoglycerides (E 472e), sorbitan monostearate (E 491).

Suitable antioxidants and substances which can intensify the antioxidative effect are the naturally occurring tocopherols and the derivatives thereof, tocotrienols, flavonoids, ascorbic acid and the salts thereof, alpha-hydroxy acids (for example citric acid, lactic acid, malic acid, tartaric acid) and the Na, K and Ca salts thereof, ingredients isolated from plants, extracts or fractions thereof, for example from tea, green tea, algae, grape seeds, wheat germs, rosemary, oregano, flavonoids, quercetin, phenolic benzylamines. Also suitable as antioxidants are propyl gallate, octyl gallate, dodecyl gallate, butylhydroxyanisol (BHA), butylhy-droxytoluene (BHT), lecithins, monoglycerides and diglycerides of edible fatty acids esterified with citric acid, orthophosphates and Na, K and Ca salts of monophosphoric acid and ascorbyl palmitate.

The W/O emulsions according to the invention are particularly suitable for application to food product surfaces, the food products having preferably a water content of at most 10% by weight, preferably of at most 5% by weight. In a preferred embodiment, the W/O emulsion according to the invention at application temperature has a sufficiently low viscosity to allow application of the W/O emulsion by means of spraying. Preferred food products, to the surfaces of which a W/O emulsion according to the invention can be applied, include for example crackers, chips (for example based on potatoes, corn (maize), cereal or bread), extruded nibbles (snacks, for example flips) or pretzel-like baked goods (for example salt sticks). W/O emulsions according to the invention are typically applied to the food product surfaces in an amount of 0.5 to 6 wt.%., based on the total weight of the food product.

As mentioned hereinbefore, one aspect of the present invention relates to the use of a compound of the above-defined Formula (I) or of a mixture of compouns of Formula (I) for producing, imparting, modifying or intensifying an umami flavor.

Preferably, the compounds of Formula (I) (in a sensorially effective amount), their flavoring mixtures or the compositions for oral consumption according to the invention are used in (i) ready-to-use or ready-to-eat products or (ii) semifinished products used for nutrition, or consumption for pleasure, in particular in sodium glutamate-reduced or sodium glutamate-free products used for nutrition, or consumption for pleasure. In this respect, that which was stated hereinbefore applies accordingly.

The term "sodium glutamate-reduced" refers in this case to the fact that the ready-to-use or ready-to-eat product or the semifinished product according to the invention contains much less sodium glutamate than is contained in the corresponding conventional products. The sodium glutamate content is in this case about 5 to 98% by weight, preferably 10 to 50% by weight, particularly preferably 20 to 50% by weight below the sodium glutamate content of the conventional products. If, in addition to one or more compounds of Formula (I), sodium glutamate is also present in a ready-to-use or ready-to-eat product or semifinished product according to the invention, the ratio by weight of the total amount of compounds of Formula (I) to sodium glutamate lies preferably in the range of from 1 : 1 to 1 : 200, more preferably in the range of from 1 : 2 to 1 : 100, even more preferably in the range of from 1 : 5 to 1 : 50.

A ready-to-eat or ready-to-use product according to the present invention preferably comprises a total amount of the compounds of Formula (I) as defined above in the range of from 1 ppm to 1000 ppm, preferably of from 2 ppm to 750 ppm, more preferably of from 4 ppm to 500 ppm, even more preferably of from 5 ppm to 250 ppm, particularly preferably of from 8 ppm to 200 ppm, most preferably of from 10 ppm to 100 ppm,
and preferably no sodium glutamate or a content of from 0.00001 to 2% by weight, preferably 0.001 to 1% by weight, more preferably 0.01 % by weight to 0.25%, in particular 0.025% by weight to 0.1% by weight of sodium glutamate,
in each case based on the total weight of the product.

A semifinished product according to the present invention preferably comprises a total amount of the compounds of Formula (I) as defined above in the range of from 50 ppm to 50000 ppm (corresponding to 5 wt.%), preferably of 75 ppm to 25000 ppm (corresponding to 2.5 wt.%), more preferably of from 100 ppm to 10000 ppm, particularly preferably of from 125 ppm to 5000 ppm,
and preferably no sodium glutamate or a content of from 0.001 to 10% by weight, preferably 0.01 to 5% by weight, in particular 0.1% by weight to 2% by weight of sodium glutamate,
in each case based on the total weight of the semifinished product.

A semifinished product according to the present invention optionally comprises a content of from 0.0001% by weight to 40% by weight, preferably 0.001% by weight to 25% by weight of an aroma composition, based on the total weight of the semifinished product.

Particularly relevant are sodium glutamate-reduced compositions or products according to the invention comprising sodium glutamate, wherein the amount of sodium glutamate is not sufficient to be perceived in a comparative composition or product, which is free of a compound of Formula (I) according to the invention but is otherwise identical in its composition, as a satisfactory umami flavor; the amount of the mixture according to the invention is sufficient to achieve a satisfactory umami flavor impression.

If a ready-to-eat or ready-to-use product according to the present invention contains one or more additional ingredients selected from the group of capsaicin, dihydrocapsaicin, nordihydrocapsaicin, chavicine, piperine, and allyl isothiocyanate, the total amount of these additional ingredients preferably is 0.01 ppm or more, more preferably is 0.05 ppm or more, more preferably 0.1 ppm or more, even more preferably 0.5 ppm or more, particularly preferably 1 ppm or more, and most preferably 2 ppm or more, in each case based on the total weight of the ready-to-eat or ready-to-use product.

Ready-to-eat or ready-to-use products (typically used for nutrition, or consumption for pleasure) according to the present invention) are in particular baked goods (for example bread, dry biscuits, cakes, other pastries), beverages (for example vegetable juices, vegetable juice products), instant beverages (for example instant vegetable beverages), meat products (for example ham, fresh sausage or uncured sausage products, spiced or marinated fresh or cured meat products), eggs or egg products (dried egg, egg white, egg yolk), cereal products (for example precooked ready-to-eat rice products, rice flour products, millet and sorghum products, uncooked or precooked noodles and pasta products), dairy products (for example cream cheese, soft cheese, hard cheese, milk beverages, whey, butter, products containing partially or completely hydrolyzed milk protein), products made of soy protein or other soy bean fractions (for example soy milk and products produced therefrom, soy lecithin-containing products, fermented products such as tofu or tempeh or products produced therefrom, soy sauces), vegetable products (for example ketchup, sauces, dried vegetables, deep-frozen vegetables, precooked vegetables, vegetables pickled in vinegar, vegetable concentrates or pastes, vegetable preserves, potato products), nibbles (for example baked or fried potato chips or potato dough products, bread dough products, corn (maize), rice or peanut-based extrudates), fat and oil-based products or emulsions thereof (for example mayonnaise, remoulade, dressings, seasoning products), other ready meals and soups (for example dried soups, instant soups, precooked soups), sauces (instant sauces, dried sauces, ready-to-eat sources), spices or spiced products (for example mustard products, horseradish products), seasoning mixes and in particular seasonings used for example in the snack sector.

Particularly preferred are semifinished products (preferably having a reduced sodium glutamate content) used for nutrition, or consumption for pleasure, for example baked goods (for example bread, dry biscuits, cakes, other pastries), vegetable juice products, meat products (for example ham, fresh sausage or uncured sausage products, spiced or marinated fresh or cured meat products), eggs or egg products (dried egg, egg white, egg yolk), cereal products (for example precooked ready-to-eat rice products, uncooked or precooked noodles and pasta products), dairy products (for example cream cheese, soft cheese, hard cheese, milk beverages, whey, butter, products containing partially or completely hydrolyzed milk protein), products made of soy protein or other soy bean fractions (for example soy milk and products produced therefrom, soy lecithin-containing products, fermented products such as tofu or tempeh or products produced therefrom, soy sauces), fish sauces such as for example anchovy sauces, oyster sauces, vegetable products (for example ketchup, sauces, dried vegetables, deep-frozen vegetables, precooked vegetables, vegetables pickled in vinegar, vegetable preserves, potato products), nibbles (for example baked or fried potato chips or potato dough products, bread dough products, corn or peanut-based extrudates), fat and oil-based products or emulsions thereof (for example mayonnaise, remoulade, dressings, seasoning products), ready meals, soups (for example dried soups, instant soups, precooked soups), stock cubes, sauces (instant sauces, dried sauces, ready-to-eat sources), spices, relishes, condiments, seasoning mixes and in particular seasonings used for example in the snack sector.

The products in the sense of the invention can also be in the form of capsules, tablets (non-coated and coated tablets, for example gastric juice-resistant coatings), dragées, granules, pellets, solid mixes, dispersions in liquid phases, as emulsions, as powders, as solutions, as pastes or as other swallowable or chewable products, for example as dietary supplements.

The semifinished products according to the invention are generally used to produce ready-to-use or ready-to-eat products (typically used for nutrition, or consumption for pleasure).

In particular, semifinished products according to the invention can serve additively to intensify the umami flavor of sodium glutamate-reduced food and luxury food products and also directly as condiments for the industrial or non-industrial preparation of food and/or luxury food products.

The compositions and products according to the invention are preferably produced in that the compounds of Formula (I) or the mixtures thereof are dissolved and mixed in mixtures of ethanol and optionally demineralized and/or purified water; subsequently, the solutions are transformed into an (at least almost) solid formulation by a drying process, preferably a spray drying, vacuum freeze drying, reverse osmosis, evaporation or other concentration process or a combination of the aforementioned processes. In this case, the drying can be carried out using excipients (for example starches, starch derivatives, maltodextrins, silica gel, celluloses, see above). Preferably, the drying is carried out by means of spray drying or vacuum freeze drying.

Preferred compositions and products according to the invention are relishes, seasoning mixes, condiments, stock cubes, instant soups, instant sauces, vegetarian (ready-to-eat) meals, meat-containing (ready-to-eat) meals, fish sauces such as for example anchovy sauces, oyster sauces and soy sauces.

According to a further preferred embodiment, for producing compositions and products according to the invention, compounds of Formula (I) or mixtures thereof and optionally other constituents are first incorporated in emulsions, in liposomes (for example starting from phosphatidylcholine), in microspheres, in nanospheres or else in capsules, granules or extrudates made up of a matrix suitable for food and luxury food products (for example of starch, starch derivatives, cellulose or cellulose derivatives such as hydroxypropyl cellulose, other polysaccharides such as alginate, natural fats, natural waxes such as beeswax or carnauba wax or of proteins such as gelatin).

In a further preferred production method, compounds of Formula (I) or mixtures thereof are used with one or more suitable complexing agents, for example complexed with cyclodextrins or cyclodextrin derivatives, preferably alpha or beta-cyclodextrin, and used in this complexed form.

Particularly preferred are products according to the invention in which the matrix is selected in such a way that the compounds of Formula (I) or mixtures thereof are released from the matrix in a delayed manner, thus providing a long-lasting effect. Examples of the matrix used in this case include natural fats, natural waxes (for example beeswax, carnauba wax) or else natural bulking substances (wheat fibres, apple fibres, oat fibres, orange fibres).

In the case the compounds of Formula (I) of the present invention are used in animal or pet food, these can advantageously be incorporated into and be part of vertebrate food, preferably food for pigs, cows, horses, sheep, goats, chicken, turkeys, rodents (preferably mice, rats, guinea pigs and hamsters), dogs and cats since these vertebrates are known to perceive "umami" tasting compounds. Preferred animal food is dog food, cat food, pig food, rat food and mouse food. Said animal food preferably comprises one or more amino acids, preferably one or more amino acids of constituent (B-5) defined above and glutamic acid and/or MSG.

Pig food comprising the compounds of Formula (I), a flavoring mixture according to the present invention or a composition for oral consumption according to the present invention, or pig food as a ready-to-eat or ready-to-use product according to the present invention, or as semifinished product according to the present invention preferably comprises asparagine, alanine, serine, 4-hydroxyproline, threonine, glutamic acid and/or MSG.

Cat food comprising the compounds of Formula (I), a flavoring mixture according to the present invention or a composition for oral consumption according to the present invention, or cat food as a ready-to-eat or ready-to-use product according to the present invention, or as semifinished product according to the present invention preferably comprises proline and/or taurine (as part of constituent (B-5) defined above), and optionally additionally nepetalactone and/or catnip.

Mouse food comprising the compounds of Formula (I), a flavoring mixture according to the present invention or a composition for oral consumption according to the present invention, or mouse food as a ready-to-eat or ready-to-use product according to the present invention, or as semifinished product according to the present invention preferably comprises methionine, cysteine, arginine and/or threonine.

Further constituents of a ready-to-eat product, a ready-to-use product or semifinished product according to the invention used for nutrition, or consumption for pleasure can be conventional primary materials, additives and auxiliaries for food or luxury food products, for example water, mixtures of fresh or processed, vegetable or animal primary or raw materials (for example raw, pan-fried, dried, fermented, smoked and/or boiled meat, bones, gristle, fish, vegetables, herbs, nuts, vegetable juices or pastes or mixtures thereof), digestible or non-digestible polycarbohydrates (amylose, amylopectin, inulin, xylans, cellulose), sugar alcohols (for example sorbitol, erythritol), natural or hardened fats (for example tallow, lard, palm fat, coconut fat, hardened vegetable fat), fatty acids or salts thereof (for example potassium stearate), peptides (for example glutathione), native or processed proteins (for example gelatin), enzymes (for example peptidases), nucleic acids, nucleotides, further flavor-masking agents for unpleasant flavor impressions, further flavor modulators for further, generally not unpleasant flavor impressions, other flavor-modulating substances (for example inositol phosphate or 2-phenoxypropionic acid), emulsifiers, stabilizers, preservatives (for example benzoic acid and salts thereof, sorbic acid and salts thereof), antioxidants (for example tocopherol, ascorbic acid), chelators (for example citric acid), organic or inorganic acidifying agents (for example acetic acid, phosphoric acid), additional bitter substances (for example quinine, caffeine, limonin, amarogentin, humulones, lupulones, catechins, tannins), substances preventing enzymatic browning (for example sulfite, ascorbic acid), (further) essential oils, plant extracts, and natural or synthetic colorants or coloring pigments (for example carotenoids, flavonoids, anthocyans, chlorophyll and derivatives thereof).

Preferably, compositions or products according to the invention additionally contain an aroma composition comprising two or more, more preferably four or more, even more preferably six or more aroma substances in order to further complement and refine the flavor and/or the smell of a composition or product according to the invention. Suitable aroma compositions contain for example synthetic, natural or nature-identical aroma substances, fragrances and flavoring substances, reaction flavors, or the like.

Additional aroma and/or flavor modulating substances are preferably selected from the group consisting of adenosine-5'-monophosphate, cytidine-5'-monophosphate, inosine-5'-monophosphate, and the physiologically acceptable salts thereof, lactisole and its physiologically acceptable salts, 2,4-dihydroxy-benzoic acid, 3-hydroxybenzoic acid, sodium salts, preferably sodium chloride, sodium lactate, sodium citrate, sodium acetate, and sodium gluconate.

According to another embodiment of the present invention, a composition, ready-to-use or ready-to-eat product or semifinished product in each case as described above additionally comprises one or more sweet tasting substances, preferably one or more mono- or discaccharide, one or more sugar alcohols and/or one or more sweeteners.

If the compounds according to the invention of the Formula (I) or the mixtures thereof are used in combination with one or more mono- or discaccharides, one or more mono- or discaccharides are preferably selected from the group consisting of sucrose (= saccharose), lactose, glucose, maltose, fructose, maltose, glucose syrup, and high fructose corn sirup.

If the compounds according to the invention of the Formula (I) or the mixtures thereof are used in combination with one or more sugar alcohols, one or more sugar alcohols are preferably selected from the group consisting of erythritol, sorbitol, xylitol, lactitol, mannitol, dulicitol, fucitol, maltitol, isomalt, and glycerol.

If the compounds according to the invention of the Formula (I) or the mixtures thereof are used in combination with one or more sweetener, one or more sweeteners are preferably selected from the group consisting of sodium cyclamate, acesulfame K, neohesperidin dihydrochalcone, saccharin, saccharin sodium salt, aspartame, superaspartame, neotame, alitame, sucralose, magap, lugduname, carrelame, sucrononate, sucrooctate, miraculin, curculin, monellin, mabinlin, thaumatin, curculin, brazzein, pentadin, or extracts or fractions thereof obtained from natural sources containing said amino acids and/or proteins, neohesperidin dihydrochalcone, steviolgylcoside, stevioside, steviolbioside, rebaudiosides (preferably rebaudioside A, rebaudioside B, rebaudioside C, rebaudioside D, rebaudioside E, rebaudioside F, rebaudioside G, rebaudioside H, dulcoside, rubusoside), suavioside A, suavioside B, suavioside G, suavioside H, suavioside I, suavioside J, baiyunoside 1, baiyunoside 2, phlomisoside 1, phlomisoside 2, phlomisoside 3, phlomisoside 4, abrusoside A, abrusoside B, abrusoside C, abrusoside D, cyclocaryoside A and cyclocaryoside I, oslandin, polypodoside A, strogin 1, strogin 2, strogin 4, selligueanin A, dihydroquercetin-3-acetate, perillartine, telosmoside A₁₅, periandrin I-V, pterocaryoside, cyclocaryoside, mukurozioside, bryoside, bryonoside, bryonodulcoside, carnosifloside, scandenoside, gypenoside, trilobatin, phloridzin, dihydroflavanol, hematoxylin, cyanin, chlorogenic acid, albiziasaponin, telosmoside, gaudichaudioside, mogroside, hernandulcine, monatin, glycyrrhetin acid, glycyrrhizin, phyllodulcin, or the physiologically acceptable salts thereof, preferably the respective potassium, sodium, calcium or ammonium salts thereof.

A flavoring mixture according to the present invention, a composition for oral consumption according to the present invention, a ready-to-eat product according to the present invention, a ready-to-use product according to the present invention (in particular an oral care product), a semifinished product for oral use according to the present invention and a semifinished oral consumption according to the present invention as defined hereinbefore respectively may preferably additionally comprise one or more additional physiological cooling agents, and thereby may lead to modified, enhanced and/or intensified physiological cooling effects, preferably said one or more further physiological cooling agents are selected from the group consisting of menthol (preferably L-menthol, D-menthol, racemic menthol, isomenthol, neoisomenthol, neomenthol), isomenthone, menthone, menthone derivatives (preferably L-menthone glycerol ketal), p-menthane-3,8-diol, cubebol, isopulegol and its esters (preferably L-(-)-isopulegol, L-(-)-isopulegol acetate), menthyl ethers (preferably (L-menthoxy)-1,2-propanediol, (L-menthoxy)-2-methyl-1,2-propanediol, L-menthyl-methyl ether), menthyl esters (preferably menthyl formate, menthyl acetate, menthyl isobutyrate, menthyl lactate, L-menthyl-L-lactate, L-menthyl-D-lactate, L-menthyl-(2-methoxy)acetate, L-menthyl-(2-methoxyethoxy)acetate, L-menthyl pyroglutamate), menthyl carbonates (preferably L-menthyl propylene glycol carbonate, L-menthyl ethylene glycol carbonate, L-menthyl glycerol carbonate or mixtures thereof), semi-esters of menthols with a dicarboxylic acid or derivatives thereof (preferably menthyl oxamate, menthyl-N-methyloxamate, menthyl-N-ethyloxamate, mono-L-menthyl succinate, mono-L-menthyl glutarate, mono-L-menthyl malonate, O-L-menthyl succinic acid ester-N,N-(dimethyl)amide, O-L-menthyl succinic acid ester amide), 2,3-dimethyl-2-(2-propyl)-butanoic acid derivatives (preferably 2,3-dimethyl-2-(2-propyl)-butanoic acid-N-methyl amide [WS-23]), menthane carboxylic acid amides (preferably L-menthane carboxylic acid-N-ethyl amide [WS-3], N^{α}-(L-menthanecarbonyl)glycine ethyl ester [WS-5], menthane carboxylic acid-N-(4-methoxyphenyl)-amide [WS-12], L-menthane carboxylic acid-N-tert-butyl amide [WS-14], L-menthane carboxylic acid-N-(4-cyanophenyl)amide, N-(4-cyanomethylphenyl) p-menthanecarboxamide), L-menthane carboxylic acid-N-(alkoxyalkyl)amides, L-menthane carboxylic acid-N-(alkylthioalkyl)amides, and pyrrolidone derivatives of cycloalkyldione derivatives (preferably 3-methyl-2(1-pyrrolidinyl)-2-cyclopenten-1-one).

A flavoring mixture according to the present invention, a composition for oral consumption according to the present invention, a ready-to-eat product according to the present invention, a ready-to-use product according to the present invention, a semifinished product for oral use according to the present invention and a semifinished oral consumption according to the present invention as defined hereinbefore respectively may preferably additionally comprise one or more further agents for masking or reducing an unpleasant flavor impression. Preferrd additional masking agents are selected from the group of substances (further) reducing or eliminating undesired and unpleasant off-notes, in particular bitter, cardboardy, metallic, and/or rancid notes.

It will be clear from the foregoing that a further aspect of the present invention also relates to a method for producing, imparting, modifying or intensifying a flavor, in particular an umami flavor, in a (i) ready-to-use or ready-to-eat product or (ii) semifinished product for oral use or oral consumption, preferably used for nutrition, oral care, or consumption for pleasure.

Thus, the present invention also relates to a method for imparting or intensifying a sensory effect, in particular for
(a) imparting or intensifying an umami flavor and/or a physiological cooling effect,
   and/or
(b) intensifying one, several or all of the sensory impressions mouthfeel, meaty, roasty, salty, mouth-watering and long-lastingness of other orally consumable constituents, preferably of other orally consumable constituents of (i) a ready-to-use or ready-to-eat product or (ii) a semifinished product for oral use or oral consumption according to the present invention as defined hereinbefore,
   including the following steps:
   1.) mixing a sensorially effective amount of a compound or mixture of compounds according to the present invention, a flavoring mixture according to the present invention, or a composition for oral consumption according to the present invention, with one or more further orally consumable constituents, preferably one or more further orally consumable constituents of (i) a ready-to-eat or ready-to-use product or (ii) a semifinished product, preferably one or more further orally consumable constituents of (i) a ready-to-eat or ready-to-use product or (ii) a semifinished product according to the present invention,
      or
      applying a sensorially effective amount of a compound or mixture of compounds according to the present invention, a flavoring mixture according to the present invention, or a composition for oral consumption according to the present invention, to (i) a ready-to-eat or ready-to-use product or (ii) a semifinished product, preferably (i) a ready-to-eat or ready-to-use product or (ii) a semifinished product according to the present invention,
      and optionally,
   2.) orally consuming the product obtained in step 1.).

The flavoring mixtures, compositions for oral consumption, ready-to-use or ready-to-eat products and semifinished products according to the present invention are easily prepared using conventional methods well-known in the art.

The present invention also relates to a method for producing
- a flavoring mixture according to the present invention as defined above, preferably in one of the preferred or particularly preferred embodiments, or
- a composition for oral consumption according to the present invention as defined above, preferably in one of the preferred or particularly preferred

embodiments,
- (i) a ready-to-use or ready-to-eat product or (ii) a semifinished product for oral use or oral consumption according to the present invention (as defined above),
   including the following step:
   mixing a compound of Formula (I) as defined above with
      or
      applying a compound of Formula (I) as defined above to
      or
   embedding a compound of Formula (I) in an enveloping or matrix material and subsequently mixing the resulting material with or applying the resulting material to
   one, several or all further constituents of the said flavoring mixture, the said composition for oral consumption or the said product.

In this case, that which was stated hereinbefore applies accordingly with regard to preferred flavoring mixtures, compositions for oral consumption, ready-to-use or ready-to-eat products and semifinished products for oral use or oral consumption.

The sensory effects described above in detail were also perceived in the application examples described hereinbelow.

### Examples

The following examples illustrate the present invention. Unless explicitly stated otherwise, all particulars (especially percentages and amounts) relate to the weight.

The ¹H-NMR spectra are recorded in solution of CDCl₃ or DMSO-d₆ with a Bruker 300 MHz spectrometer. The chemical shifts are defined as δ with CDCl₃ or DMSO-d₆ and D₂O as internal standards.

The HPLC/MS analyses are performed with a Gilson instrument by utilizing a X-Terra RP18 column (5 mm, 4.6x50 mm) coupled to an UV detector (220 nm) and a Finnigan Aqa mass spectrometer (electron spray, positive ionization mode). General conditions utilized for the analyses: flow: 1.2 mL/min; column temperature: 50°C; A/B elution gradient (eluent A: 0.1% formic acid in water; eluent B: 0.1% formic acid in acetonitrile): 5-95% of B from 0 to 8.0 minutes, 95% of B from 8.0 to 9.5 minutes.

Abbreviations which are used in the description of the Schemes and the Examples that follows are:
EtAc: ethyl acetate
TEA: Triethylamine
DCM: dichloromethane
CC: Column Chromatography
RT: Room Temperature
NMR: Nuclear Magnetic Resonance
ESI: Electrospray ionization

### Synthesis Example 1: imidazo[1,2-a]pyridine-2-ylmethyl 4-(oxolan-2-ylmethoxy)benzoate

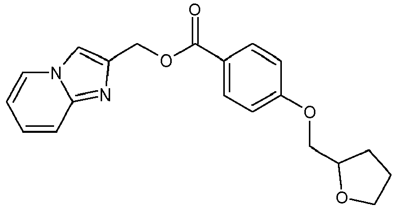

Formula: C₂₀H₂₀N₂O₄

MW: 352.38

Mass/charge ratio: 353.22 (MH+, ESI pos., 3.2KV, 25V, 350°C)

¹H-NMR (300 MHz, DMSO-d₆) δppm 1.60-2.05 (m, 4H); 3.65-3.89 (m, 2H); 4.11 (m, 2H); 4.22 (dt, 1H); 5.55 (s, 2H); 6.82 (m, 2H); 7.30 (dd, 2H), 7.54 (ddd, 2H), 8.11 (dt, 2H), 8.59 (dd, 1H).

The above compound is synthesized according to Scheme 1

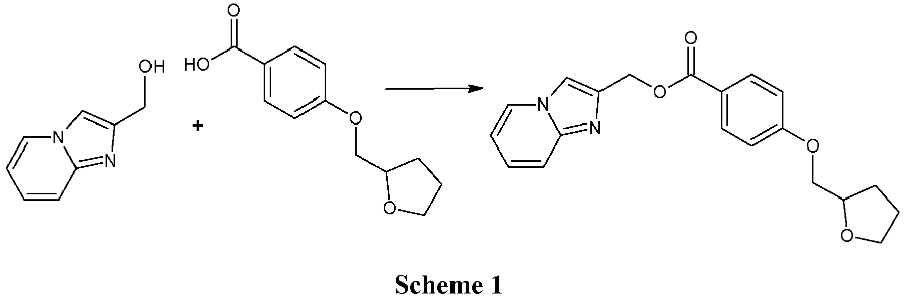

4-(oxolan-2-ylmethoxy)benzoic acid (RN 565194-75-4; Aldrich)(1 mmol) was suspended in dry toluene (12 mL), then trifluoroacetic anhydride (1.2 mmol) was added under a nitrogen atmosphere and the suspension stirred until complete solubilization occurred at RT. Imidazo[1,2-a]pyridin-2-ylmethanol (RN82090-52-6; Aldrich)(1.2 mmol) was added, the solution was stirred for 2h at RT and then diluted with EtAc (8 mL), washed with a 2M NaOH solution (2x5 mL) and then with brine (2x5 mL). The organic phase was dried over Na₂SO₄ and the solvent evaporated to obtain the crude compound which was purified by CC (Cyclohexane/EtAc 9:1) to give the desired compound as a whitish powder.
(Yield: 81%).

Analogously, using the corresponding acid and alcohol the following compounds were obtained:

### Synthesis Example 2: 6-chloroimidazo[1,2-a]pyridine-2-ylmethyl 2-(4-methylbenzoyl)benzoate

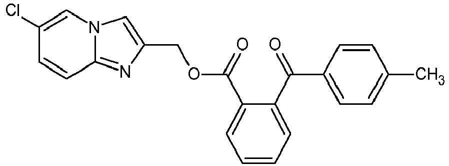

Formula: C₂₃H₂₇ClN₂O₃

MW: 404.85

Mass/charge ratio: 405.71 (MH+, ESI pos., 3.2KV, 25V, 350°C)

¹H-NMR (300 MHz, DMSO-d₆) δppm 2.38 (s, 3H); 5.50 (s, 2H); 7.25 (m, 1H); 7.34 (q, 1H); 7.50 (dd, 1H); 7.61 (dd, 1H); 7.71-7.89 (m, 2H); 8.04 (d, 1H); 8.78 (s, 1H).

The above compound is synthesized according to the procedure described in Scheme 1, using (6-chloroimidazo[1,2-a]pyridin-2-yl)methanol (RN1039416-36-8; Fluorochem) as an alcohol and 2-(4-methylbenzoyl)benzoic acid (RN85-55-2; Alfa Aesar) as an acid. (Yield: 65%)

### Synthesis Example 3: imidazo[1,2-a]pyridine-2-ylmethyl 2-(methylsulfanyl)pyridine-4-carboxylate;

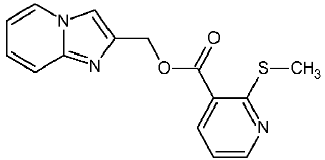

Formula: C₁₅H₁₃N₃O₂S

MW: 299.35

Mass/charge ratio: 300.23 (MH+, ESI pos., 3.2KV, 25V, 350°C)

¹H-NMR (300 MHz, DMSO-d₆) δppm 2.71 (s, 3H); 5.33 (s, 2H); 7.15-7.27 (m, 2H); 7.50 (dd, 1H); 7.69 (s, 1H), 8.31 (dd, 1H), 8.45 (ddd, 2H), 8.62 (dd, 1H).

The above compound is synthesized according to the procedure described in Scheme 1, using 2-(methylsulfanyl)pyridine-3-carboxylic acid (RN74470-26-1; Aurora) as an acid (Yield: 55%)

### Synthesis Example 4: 4-acetyl-N-((6-chloroimidazo[1,2-a]pyridine-2-yl)methyl)-N-methylbenzene- I -sulfonamide

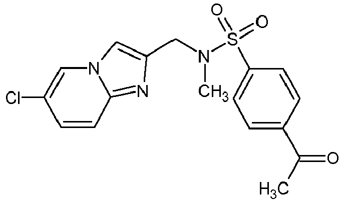

The above compound is synthesized according to Scheme 2.

Formula: C₁₇H₁₆ClN₃O₃S

MW: 377.84

Mass/charge ratio: 378.71 (MH+, ESI pos., 3.2KV, 25V, 350°C)

¹H-NMR (300 MHz, DMSO-d₆) δppm 2.42 (s, 3H); 2.61 (s, 3H); 4.71 (s, 2H); 7.44-7.52 (m, 3H); 7.91 (m, 2H); 8.14 (m, 2H); 8.64 (s, 1H).

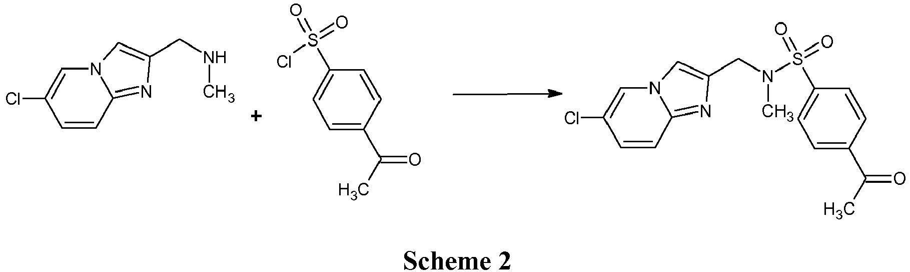

To a suspension of 1-(6-chloroimidazo[1,2-a]pyridin-2-yl)-N-methylmethanamine (RN554407-29-3; Akos)(0.16 mmol) stirred in DCM, TEA (0.19 mmol) was added, followed by the addition of neat 4-acetyl-benzenesulfonyl chloride (RN1788-10-9; Alfa Aesar). The reaction was allowed to stir at RT for 2h then treated with PS-tris-amine resin (100 mg). The reaction was allowed to stand for 15 min at RT after which the resin was removed by filtration. The filtrate was concentrated under vacuum to afford the crude product. The crude residue was purified by CC (Cyclohexane/EtAc 7:3) to afford the desired product as an off-white powder (Yield: 92%).

### Sensory Test 1: Individual compound of Formula (I)

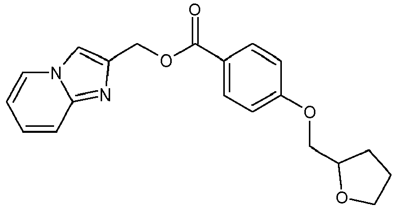

An expert panel (8 panelists) evaluated the sensory properties of the compound of Formula (I) of Synthesis Example 1. The compound of Formula (I) was first dissolved in ethanol and then added in the corresponding amount in water. The aqueous solution was evaluated by sipping, holding it in the mouth for 10 seconds, and then spitting it out.

The sensory effects observed by the expert panel for compound (I) are present in the following table.

| **Compound (I) of Synthesis Example 1** | **Description of sensory effects** |
|---|---|
| 3 ppm | Weakly umami, |
| 30 ppm | Umami, meaty, slightly salty |
| 3 ppm + 0.05 wt.% MSG | Umami, meaty, amino acid like, mouth-watering |
| 30 ppm + 0.05 wt.% MSG | Umami, meaty, salt enhancing |

### Sensory Test 2: Compounds of Formula (I)

A group of 16 trained panelists was instructed to evaluate the taste of a broth (bouillon) containing 0.5 wt.% of beef extract (Ref-beef) according to eight different descriptors in several repetitions. In a next step, these panelists were asked to rate said reference beef broth "Ref-beef" (presented at each testing session as a reference) against the following two different compositions consisting of "Ref-beef" and additionally a specific amount of the respective to be tested substance:

"Beef-I" = "Ref-beef" + 10 ppm of the compound of Formula (I) of Synthesis Example 1,

"Beef-MSG" = "Ref-beef" + 500 ppm (0.05 wt.%) of MSG

"Beef-MSG" was used as a positive umami standard.

The panelists judged the strength / intensity of the eight different descriptors in each case by awarding scores on a scale of from 0 (not corresponding to descriptor) to 6 (very strong). The results are present in the following table. All data are the averaged values for the group of 16 trained panelists for the respective scores for each descriptor.

| **Descriptor** | **Ref-Beef** | **Beef- I (10 ppm of the compound of Formula (I) of Synthesis Example 1)** | **Beef-MSG (500 ppm MSG)** |
|---|---|---|---|
| Mouthfeel | 3.5 | 5.3 | 4.8 |
| Salty | 2.1 | 3.4 | 2.5 |
| Meaty | 4.1 | 5.3 | 5.3 |
| Mouth-watering | 3.3 | 4.5 | 4.4 |
| Roasty | 3.4 | 3.6 | 3.8 |
| Sweet | 1.0 | 1.7 | 2.1 |
| Sour | 2.7 | 2.8 | 2.9 |
| Long-lastingness | 3.4 | 5.2 | 4.3 |

### Application Example 1: Spray-dried powders

| **Ingredient** | **A** wt.% | **B** wt.% | **C** wt.% | **D** wt.% |
|---|---|---|---|---|
| Compound of Formula (I) | 2 | 5 | 10 | 8 |
| Maltodextrin | 98 | - | 90 | - |
| Maltodextrin and gum Arabic (10:1) | - | 95 | - | Ad 100 |
| Sodium chloride | - | - | - | 5 |

The constituents are dissolved in a mixture of ethanol and demineralized water and subsequently spray-dried to give a powder.

### Application Example 2: Semi-finished products

| **Part** | **Ingredient** | **Preparation** |
|---|---|---|
| A | Compound of Formula (I) | 0.018 - 0.090 g |
| | Sodium chloride | 13 - 15 g |
| B | Yellow mustard seed flour | 3.5 - 5 g |
| | Mustard flavor | 0.1 g |
| | Wasabi flavor | 0.1 g |

The wasabi flavor and the mustard flavor each also contained an effective amount of allyl isothiocyanate. Part A is added to 300 ml of water and dissolved while stirring. The solution is further diluted with water to 1000 g and then freeze-dried. The resulting freeze-dried product is mixed with yellow mustard seed flour and the flavor(s) from part B and prepared to form a condiment.

### Application Example 3: Reaction flavors

| **Ingredient** | **Preparation A [g]** |
|---|---|
| L-alanine | 41.0 |
| L-aspartic acid | 114.0 - 123.0 |
| Succinic acid | 4.2 - 4.7 |
| Calcium chloride dihydrate | 7.0 |
| L-cysteine·HCl monohydrate | 10-11 |
| Dipotassium phosphate | 6.0 - 7.0 |
| Fructose, ground | 1.0 - 3.0 |
| L-isoleucine | 0 - 1.6 |
| Potassium chloride | 180.0 - 230.0 |
| L-leucine | 1.6 |
| L-lysine·HCl | 3.6 |
| Magnesium chloride hexahydrate | 19.0 |
| Maltodextrin | 36.0 - 56.0 |
| L-phenylalanine | 2.0 |
| L-proline | 74.0 - 84.0 |
| L-serine | 6.0 - 6.5 |
| L-threonine | 3.0 |
| L-valine | 8.0 - 9.0 |
| Water | Ad 1000 g |
| Compound of Formula (I), 20 wt.% solution in ethanol | 9.0 - 15.0 |
| Preparation D of Application Example 1 | 0 - 90.0 |

All components are mixed at 40°C and subsequently heated at 85°C for 10 minutes. After cooling to 40°C, the pH is adjusted to a value of about 5 using potassium hydroxide solution.

### Application Example 4: Hot-spicy leek-type instant cream soups

| **Ingredient** | **Preparation** |
|---|---|
| Potato starch | 20.00 g |
| Fat powder | Ad 100 g |
| Lactose | 20.00 g |
| Maltodextrin | 14.69 - 14.724 g |
| Sodium chloride | 8.00 g |
| Spinach powder | 2.00 g |
| Green leek powder, flavored | 2.00 g |
| Citric acid, powder | 0.30 g |
| Hardened vegetable fat | 3.00 g |
| Freeze-dried leek | 1.00 g |
| Chicken flavor, spray-dried | 1.00 g |
| "Hot chili"-type seasoning powder | 2.00 g |
| "Fried onion"-type seasoning mixture | 0.60 g |
| "Vegetable broth"-type yeast powder | 0.30 g |
| Curcuma extract (turmeric) | 0.070 g |
| Compound of Formula (I) | 0.015 - 0.060 g |

The "Hot chili"-type seasoning powder contained 0.35 wt% of capsaicin, dihydrocapsaicin, and nordihydrocapsaicon in a ratio of 7 : 2 : 1 by weight.

100 ml of hot water are poured onto 5 g of the powder mixture in order to obtain a ready-to-eat hot-spicy leek-type cream soup.

### Application Example 5: Instant chicken soups with noodles

| **Ingredient** | **Preparation** |
|---|---|
| Starch | 16.00 g |
| Sodium chloride | 5.00 - 7.00 g |
| Saccharose, refined | 3.20 g |
| Sodium glutamate (MSG) | 0 - 0.025 g |
| Sodium inosinate / sodium guanylate (1:1) | 0.60 - 0.80 g |
| Acid-hydrolyzed plant protein | 8.00 g |
| Fat powder | 2.00 g |
| Vegetable fat, spray-dried | 1.00 g |
| Freeze-dried chicken, in pieces | 2.00 g |
| Soup noodles | Ad 100 g |
| Maltodextrin | 14.12 - 15.354 g |
| Chinese vegetables, freeze-dried | 4.60 g |
| Chicken flavor | 8.00 g |
| Riboflavin (colorant) | 0.040 g |
| Compound of Formula (I) | 0.015 - 0.060 g |
| Preparation of Application Example 2 | 0 - 2.10 g |

4.7 g of the powder mixture are boiled for 10 minutes in 100 ml of water in order to obtain a ready-to-eat soup.

### Application Example 6: Pepper-type seasoning mixtures

| **Ingredient** | **Preparation** |
|---|---|
| Milk protein | 0.80 g |
| Carob flour | 2.00 g |
| Corn starch | Ad 100 g |
| Sodium chloride | 14.00 g |
| Paprika powder | 13.00 g |
| Tomato powder | 13.00 g |
| Saccharose | 4.00 g |
| Garlic powder | 0.50 g |
| Hardened vegetable fat | 8.00 g |
| Fat powder | 11.00 g |
| Beetroot and paprika food colorants | 2.00 g |
| Mixed peppers flavor | 2.00 g |
| "Herb de Provence" spice mixture | 1.20 g |
| Tomato flavor | 0.40 g |
| Black pepper extract (containing piperine and chavicine) | 0.10 - 0.20 g |
| Compound of Formula (I) | 0.055 - 0.40 g |

Each 100 g of a mixture of equal amounts of diced chicken and pork meat are seasoned uniformly with 2.5 g of the preparation and then pan-fried.

### Application Example 7: Seasoning mixtures for chips

| **Ingredient** | **Seasoning** |
|---|---|
| Sodium glutamate | 2.00 g |
| Cheese powder | 10.00 g |
| Garlic powder | 2.00 g |
| Whey powder | Ad 100 g |
| Seasoning extract oil | 0.20 g |
| Paprika powder | 9.80 g |
| Sodium chloride | 21.00 g |
| Tomato powder | 9.00 g |
| Dry flavor | 2.50 g |
| Silicon dioxide | 0.02 g |
| Vegetable oil | 0.02 g |
| Onion powder | 3.00 g |
| Cream flavor concentrate | 0.03 g |
| Cheese flavor | 0.03 g |
| Tomato flavor concentrate | 0.04 g |
| Compound of Formula (I) | 0.030 - 0.075 g |
| Preparation from Application Example 6 | 0 - 2.10 g |

5 g of the seasoning are sprinkled onto 95 g of potato chips.

7 g of the seasoning are spread evenly onto 93 g of corn tortilla chips.

### Application Example 8: Brown gravy mixtures

| **Ingredient** | **Mixture** |
|---|---|
| Corn starch | Ad 100 g |
| Maltodextrin (18 DE) (ex tapioca) | 33.10 g |
| Sodium chloride | 6.00 g |
| Caramel colour, spray-dried | 5.00 g |
| Yeast extract powder | 3.00 g |
| Sodium glutamate (MSG) | 0 - 1.30 g |
| Sugar (sucrose) | 0.50 g |
| Fat powder | 5.00 g |
| Tomato powder | 3.00 g |
| Natural vegetable extract | 1.00 g |
| Onion extract | 0.30 g |
| Black pepper extract | 0.10 g |
| Dry flavor | 1.00 g |
| Compound of Formula (I) | 0.005 - 0.025 g |
| Preparation A of Application Example 3 | 0 - 0.28 g |

1000 ml of hot water are poured onto 90 g of the gravy mixture which was stirred vigorously using an egg whisk.

### Application Example 9: Sugar-free chewing gums

| **Part** | **Ingredient** | **Preparation % by weight** |
|---|---|---|
| A | Chewing gum base | 30.00 - 31.00 |
| B | Sorbitol, powdered | Ad 100 |
| | Isomalt (Palatinit^{®}) | 9.00 - 9.50 |
| | Xylitol | 2.00 |
| | Mannitol | 3.00 |
| | Aspartam® | 0.10 |
| | Acesulfam® K | 0.10 |
| | Acacia gum emulsifier | 0.30 |
| C | Sorbitol, 70% in water | 14.00 |
| | Glycerol | 1.00 |
| D | Cool cinammon flavor (essentially consisting of cinnamaldehyde (>60%), vanillin, eugenol, caryopyhllene, menthyl acetate, linalool, menthol (16%), menthone (6%), menthyl lactate (5%), and WS-3) | 0.92 - 1.25 |
| | Compound of Formula (I) | 0.0008 - 0.0020 |

Parts A to D are mixed and kneaded intensively. The raw material can for example be processed in the form of strips, chiclets or pellets to form ready-to-use chewing gums.

### Application Example 10: Roasted shrimp ginger flavors

| **Ingredient** | **Preparation % by weight** |
|---|---|
| 2-Acetylpyrazine | 0.22 - 0.26 |
| 2-Methylbutanal | 0.40 - 0.50 |
| Dimethyl sulfide | 1.20 - 1.30 |
| Dimethyl disulfide | 0.60 - 0.70 |
| Dimethyl trisulfide | 1.30 - 1.50 |
| Isopropyl methyl disulfide | 0.15 |
| 4-Methylphenol | 0.10 |
| Safranal | 0-0.10 |
| 5E/Z,8Z,11Z-tetradecatrien-2-one | 0 - 0.08 |
| 1-Pyrroline | 2.90 - 3.30 |
| Pyridine | 1.25 - 1.30 |
| 2-Methylpyridine | 0.20 - 0.30 |
| 3-Methylpyridine | 0.55 - 0.60 |
| N-(2-Methylbutyl)pyrrolidone | 0.60 |
| 3-Methyl-N-(2-methylbutylidene)butanamine | 4.90 - 6.30 |
| 2-Methyl-N-(2-methylbutylidene)butanamine | 4.50 - 5.60 |
| 2-Nonanone | 0.80 - 0.90 |
| 2-Ethyl-3,5-dimethylpyrazine | 0.40 - 0.50 |
| 2,5-Dimethylpyrazine | 0.90 - 1.15 |
| 2,3,5-Trimethylpyrazine | 1.90 - 2.80 |
| [6]-Paradol | 0 - 0.25 |
| [8]-Gingerdione | 0-0.10 |
| Ginger extract | 0 - 0.25 |
| Jambu oleoresin | 0 - 0.17 |
| Zingerone | 0.30 - 0.45 |
| Propylene glycol | Ad 100 |
| Compound of Formula (I) | 0.80 - 1.75 |

### Application Example 11: Fried potato flavors

| **Ingredient** | **Flavor A parts by weight** | **Flavor B parts by weight** |
|---|---|---|
| Methanethiol | 380 | 480 |
| Methional | 320 | 320 |
| 2,3-Butanedione | 190 | 190 |
| 2-Methylpropanal | 1250 | 1550 |
| 2-Methybutanal | 2100 | 2900 |
| 3-Methylbutanal | 800 | 800 |
| (E)-2-Nonenal | 30 | 30 |
| (E,E)-2,4-Decadienal | 2000 | 2400 |
| (E,Z)-2,4-Decadienal | 125 | 125 |
| 2-Ethyl-3,5-dimethylpyrazin | 25 | 23 |
| 3-Ethyl-2,5-dimethylpyrazin | 15 | 14 |
| 2,3-Diethyl-5-methylpyrazin | 20 | 20 |
| Spilanthol | 25 | - |
| Black pepper extract | - | 15 |
| 3-Isobutyl-2-methoxypyrazin | 10 | 5 |

All ingredients of Flavor A are dissolved in sunflower oil such that the final amount of Flavor A in sunflower oil was 26 ppm. Thereto, a compound of Formula (I) is added in amount of 11 ppm. The resulting flavored sunflower oil is used together with corn oil to prepare a mayonnaise (which can also be used e.g. as dip for crackers). The final amount of Flavor A in said mayonnaise is about 15 ppm, the amount of the compound of Formula (I) is 6 ppm.

All ingredients of Flavor B are dissolved in soybean oil such that the final amount of Flavor B in soybean oil is 22 ppm. Thereto, a compound of Formula (I) is added in amount of 11 ppm. The resulting flavored soybean oil is used in combination with olive oil to prepare a margarine spread. The final amount of Flavor B in said margarine spread is about 9 ppm, the amount of the compound of Formula (I) is 4 ppm.

### Application Example 12: Beef strips with fajita marinade

A fajita marinade in dry form is prepared using maltodextrin, sodium chloride, black pepper, smoked chipotle peppers, parsley, cilantro, onion powder, garlic powder, sodium phosphate, modified corn starch, modified tapioca starch, vinegar, dextrose, hydroxypropyl methylcellulose, lime juice, citric acid, lime oil, "smoky-Mex-Flavor", yeast extract, sesame oil, soybean oil, and silicon dioxide.

The "smoky-Mex-Flavor" contained gamma-decalactone, 4-methylguaiacol, 4-ethylguaiacol, 4-vinylguaiacol, (E)-isoeugenol, acetovanillone, syringol, syringaldehyde, 4-acetosyringol, 4-acetosyringone, eugenol, cinnamaldehyde, anisole, thymol, acetoin, 2-butanone, 2-tridecanone, 12-methyl-tridecanal, (2E,6Z)-nonadienal, and (E,E)-2,4-decadienal, and 2,8% of a compound of Formula (I).

Freshly cut beef strips are pan-fried in peanut oil, then 18 g of fajita marinade in dry form and 125 ml of water are added thereto and the resulting mixture cooked further to the desired degree.

### Application Example 13: Onion Flavor

An onion flavoring mixture with leek aspects is prepared by mixing 2-trans-hexenal (0.26%), hexanal (0.15%), 3-cis-hexenol (1.45%), diallyl disulfide (5% solution in vegetable oil) (0.12%), leek oil (0.21%), allyl isothiocyanate (0.42%), methyl propyl disulfide (0.51%), foetida oil (0.65%), dimethyl trisulfide (1.28%), dipropyl disulfide (2.06%), dipropyl trisulfide (5.13%), propylene glycol (8.50%), onion oil (9.60%), a compound of Formula (I) (2.80%) and triacetin (ad 100%).

The following basic recipe for the production of crackers was used: wheat flour (Ad 100%), baking powder (1.25%), vegetable fat (6.35%), maltose syrup (2.25%), emulsifier (1.50%), ammonium bicarbonate (1.7%), dried skimmed milk powder (1.35%), fresh baker's yeast (0.6%), table salt (0.52%), and water (22.2%).

This onion flavor are added in amounts of 0.25%, 0.5%, 0.75% and 1% respectively to said basic recipe and the four different dough batches subsequently are formed as round crackers baked in an oven to give crackers having varying intensities of onion flavor.

Preferred snack articles according to the present invention are savory snacks, such as potato, rice or corn crisps, extrudates, popcorn, pretzels, and dough products baked in fat or in the oven. The application of a flavoring mixture according to the present invention to a snack article can be carried out by way of seasoning, sprayed-on oil slurry, fatty filling or dough flavoring.

### Application Example 14: Garlic flavor

A garlic flavoring mixture according to the present invention is prepared by mixing garlic oil (8.20 g), diallyl sulfide (0.32 g), diallyl disulfide (0.09 g), allyl isothiocyanate (1.03 g), allyl mercaptan (0.10 g), a compound of Formula (I) (1.55 g) and vegetable triglyceride (ad 500 g).

### Application Example 15: Shiitake flavor

A shiitake flavoring mixture according to the present invention is prepared by mixing 3,5-dimethyl-2-ethylpyrazine (0.09%), 2,6-dimethylpyrazine (0.14%), 1% methylthiopropanol in propylene glycol (0.17%), 2,5-dimethylpyrazine (0.16%), 3-octanol (0.45%), methylfurfuryl disulfide (0.45%), 0.1% o-cresol in triacetin (0.73%), diisopropyl trisulfide (1.24%), 2-hydroxy-3-methyl-cyclopent-2-enone (1.37%), 1-octen-3-ol (1.50%), dimethyl trisulfide (2.23%), 10% 2,3,5-trithiahexane in triacetin (4.55%), a compound of Formula (I) (2.90%), and vegetable oil (a mixture of sunflower oil, olive oil and canola oil (ratio by volume: 60 : 30 : 10) (Ad 100%).

### Application Example 16: Cat food supplement for daily use

A cat food supplement is produced essentially consisting of kelp (Ascophylum nodosum and Laminaria digitata), flaxseed, bee pollen complex (bee pollen, spirulina), hydrolyzed whitefish, chondroitin sulfate, glucosamine sulfate, methylsulfonyl methane, garlic, lecithin, catnip, dried fermentation products of several different microorganisms, Lactobacillus acidophilus, Bifidobacterium longum, protease, amylase, cellulase, lipase, vitamin A, vitamin B2, vitamin B3, vitamin B6, vitamin B12, calcium ascorbate, vitamin D, vitamin E, more than 30 different minerals (including calcium, potassium, magnesium, iron, iodine, selenium and zinc), and flavor (including a compound of Formula (I) in an amount of 53 ppm, based on the total weight of the cat food supplement).

Nutritional values: proteins: 9.0%, fat: 2.3%, natural fibers: 5.0%, total ash (minerals): 26%, moisture: 9%, carbohydrates 46.8%, iodine 800 ppm, iron 625 ppm, selenium 0.3 ppm, zinc 14 ppm. Distribution of the most relevant amino acids (indicated in wt.% of the total amount of amino acids present): alanine 5.5, lysine 4.6, arginine 5.5, methionine 1.4, aspartic acid 10.8, phenylalanine 3.5, cystine 1.6, cysteine 2.2, proline 6.7, hydroxyproline 3.8, glycine 6.0, serine 5.1, glutamic acid 12.4, threonine 4.9, histidine 1.5, tryptophan 2.0, isoleucine 3.5, tyrosine 3.6, leucine 5.3, valine 3.6.

Daily dosage: about 1.5 - 2 g of this cat food supplement are sprinkled on or mixed with the dry or wet cat food.

### Application Example 17: Dry cat food

A dry cat food product is manufactured from chicken, chicken products, chicken liver, corn, wheat, corn flour (gluten-free), chicken liver flavor (including a compound of Formula (I) in an amount of 16 ppm, based on the total weight of the dry cat food product), vegetable oil, fish oil, vitamin A, vitamin E, vitamin K, vitamin C, biotin (vitamin H), folic acid, calcium, copper, zinc, iodine, taurine, and proline.

Nutritional values: proteins: 31%, natural fatty oil: 12%, natural fibers: 2.5%, total ash (minerals): 6.5%, moisture: 8%, taurine 1000 mg/kg, vitamin A: 15000 IU/kg, vitamin D: 1500 IU/kg, vitamin E 100 IU/kg.

This dry cat food product contains vitamins, minerals, omega-3 fatty acids, omega-6 fatty acids, and taurine for a fully balanced nutrition to meet the daily needs of adult cats (weighing about 1 kg or more).

### Application Example 18: Creamy-spicy chicken soup

A creamy-spicy chicken soup in dry form is prepared using the following ingredients: whey protein, maltodextrin, milk protein, potato flakes, corn starch, evaporated cane juice, non fat milk, corn powder, corn flakes, butter powder, chicken, whey, onion powder, parsley leaves, yeast extract, garlic powder, safflower oil, black pepper, salt, curry Madras powder, further spices, gamma-amino butyric acid, esters of fatty acids, cellulose, creamy-spicy chicken flavor (comprising an effective amount of a compound of Formula (I)), guar gum, tricalcium phosphate, magnesium oxide, alfalfa (Medicago sativa), soy lecithin, magnesium stearate, yucca root powder, magnesium citrate, silicon dioxide, vitamin A, vitamin B5, vitamin B2, vitamin B3, vitamin B6, vitamin B9, vitamin B12, vitamin C, vitamin D3, vitamin E (mixed tocopherols), vitamin H, thiamine hydrochloride, manganese chelate, copper chelate, iodine chelate, selenomethionine, chromium amino acid chelate, molybdenum amino acid chelate, zinc oxide, alpha amylase, lactase, cellulase, lipase, protease, and papain.

60 g of said creamy-spicy chicken soup in dry form contain 4.45 g of total fat (saturated fat 1.45 g, monounsaturated fats 2 g; free of trans fats), 29.7 g carbohydrates (4.9 g sugars, 4 g dietary fibers), 20 g proteins, 20 mg cholesterol, 640 mg sodium, 260 mg potassium, vitamin A (50% DV; DV stands for Daily Value), vitamin C (40% DV), calcium (50% DV), iron (20% DV), vitamin D (80% DV), vitamin E (40% DV), thiamin (50% DV), riboflavin (60% DV), niacin (45% DV), vitamin B6 (70% DV), folate (80% DV), vitamin B12 (100% DV), biotin (45% DV), pantothenic Acid (45% DV), phosphorus (30% DV), iodine (40% DV), magnesium (30% DV), zinc (45% DV), selenium (40% DV), copper (35% DV), manganese (70% DV), chromium (80% DV), and molybdenum (60% DV).

Amino acid analysis of 60 g of said creamy-spicy chicken soup in dry form: alanine 697 mg, arginine 309 mg, aspartic acid 1524 mg, cystine/cysteine 298 mg, glutamic acid 2321 mg, glycine 284 mg, histidine 243 mg, hydroxyproline 16 mg, isoleucine 913 mg, leucine 1523 mg, lysine 1382 mg, hydroxylysine 29 mg, methionine 382 mg, phenylalanine 457 mg, proline 864 mg, serine 742 mg, threonine 946 mg, tryptophan 345 mg, tyrosine 398 mg, valine 824 mg, other amino acids about 5500 mg.

60 g of said creamy-spicy chicken soup in dry form are added to 225 ml of pre-heated water and stirred with a fork. After steeping for 2-3 minutes the soup is ready for consumption.

### Application Example 19: Italian sausage seasoning mix

The following two Italian sausage seasoning mixes (Mix A and Mix B) are prepared.

| **Ingredient** | **Mix A % by weight** | **Mix B % by weight** |
|---|---|---|
| Sicilian sea salt | - | Ad 100 |
| Sodium chloride | Ad 100 | - |
| Sucrose | 17.90 | 17.90 |
| Paprika powder | 14.90 | 14.90 |
| Garlic powder | 15.25 | 13.25 |
| Onion powder | 5.00 | 7.00 |
| Fennel seeds | 4.50 | 4.70 |
| Black pepper, ground | 4.75 | 4.75 |
| Parsley flakes, dried | 1.50 | 1.50 |
| Oregano flakes, dried | 0.60 | 0.60 |
| Anise seed, ground | 1.65 | 1.45 |
| Cayenne pepper, ground | 0.60 | 0.90 |
| Green pepper, ground | 0.35 | - |
| MSG | - | 0.45 |
| Compound of Formula (I) | 0.040 | 0.035 |

To two separate portions of each 300 g of ground porc (total fat: 20.8%) and 5 g of water, 16.5 g of above Mix A and Mix B are added, homogenized, and the resulting mixtures processed into cooked sausages.

## Claims

1. The use of a compound of Formula (I) 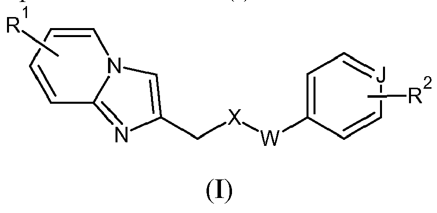 wherein:
X is O or NR³, wherein R³ is hydrogen or C₁-C₄ alkyl;
W is a carbonyl or a sulfonyl group, with the proviso that when W is a sulfonyl group, then X is NR³;
J is CH or N;
R¹ is hydrogen, halogen or C₁-C₄ alkyl;
R² is hydrogen, halogen, C₁-C₄ alkyl, C₁-C₄ alkoxy, C₁-C₄ thioalkyl, sulfonamide, NR³R⁴, wherein R³ and R⁴ are, independently from each other, hydrogen or C₁-C₄ alkyl, OCH₂R⁵, wherein R⁵ is an aromatic or heterocyclic ring;
or R² is COR⁶, wherein R⁶ is C₁-C₄ alkyl, aryl C₁-C₄ alkyl, or a phenyl ring optionally substituted with C₁-C₄ alkyl, C₁-C₄ alkoxy or halogen groups;
mixtures thereof and salts thereof suitable for human or animal consumption;
as food additive, beverage additive, and/or flavoring agent.

2. The use as defined in claim 1 of a compound of Formula (I) of claim 1,
wherein:
X is O or NR³, wherein R³ is methyl;
W is a carbonyl or a sulfonyl group;
J is CH or N;
R¹ is halogen;
R² is halogen, C₁-C₄ alkyl, C₁-C₄ alkoxy, C₁-C₄ thioalkyl, OCH₂R⁵, wherein R⁵ is phenyl or a five-membered heterocyclic ring containing at least one oxygen atom;
or R² is COR⁶, wherein R⁶ is C₁-C₄ alkyl or a phenyl ring substituted with one C₁-C₄ alkyl group.

3. The use as defined in claims 1 of a compound of Formula (I) of claim 1,
wherein:
X is O or NR³, wherein R³ is methyl;
W is a carbonyl or a sulfonyl group;
J is CH or N;
R¹ is chlorine;
R² is C₁-C₄ thioalkyl, OCH₂R⁵, wherein R⁵ is phenyl or tetrahydrofuranyl ring;
or R² is COR⁶, wherein R⁶ is methyl or a phenyl ring substituted with a methyl group.

4. The use as deined in claim 1 of a compound of Formula (I) of claim 1, which is selected from:
- imidazo[1,2-a]pyridine-2-ylmethyl 4-(oxolan-2-ylmethoxy)benzoate;
- 6-chloroimidazo[1,2-a]pyridine-2-ylmethyl 2-(4-methylbenzoyl)benzoate;
- imidazo 1,2-a]pyridine-2-ylmethyl 2-(methylsulfanyl)pyridine-4-carboxylate;
- 4-acetyl-N-((6-chloroimidazo[1,2-a]pyridine-2-yl)methyl)-N-methylbenzene-1-sulfonamide.

5. The use as defined in claim 1 as flavoring agent for
(i) producing, imparting, or intensifying an umami flavor and/or a physiological cooling effect,
and/or
(ii) enhancing, intensifying and/or rounding off one or more sensory impressions of one or more other flavoring substances, wherein preferably one, several or all of the sensory impressions mouthfeel, meaty, roasty, salty, mouth-watering and long-lastingness of said or more other flavoring substances are enhanced, intensified, and/or rounded off.

6. Flavoring mixture comprising
(A) a sensorially effective amount of a compound of Formula (I) as defined in claim 1, or of a mixture or salt thereof suitable for human or animal consumption,
and
(B) a sensorially effective amount of one or more, preferably two or more other ingredients selected from the group of substances imparting one or more sensory impressions, preferably substances imparting one or more sensory impressions selected from the group consisting of warming, heating, tingling, numbing, stinging, salivating, sulfurous, alliaceous, pungent, meat, fish, seafood, seaweed, mushroom, aromatic, smoky, tangy, spicy, herbal, savory, and mouthfeel.

7. Flavoring mixture according to claim 6, wherein one or more additional ingredients of group (B) are selected from the group consisting of constituents (B-1), (B-2), (B-3), (B-4), (B-5), (B-6), and (B-7)
(B-1) substances selected from the group consisting of physiologically warming and/or tingling sensates,
(B-2) aroma molecules imparting one or more sulfurous, alliaceous and/or pungent notes, preferably selected from the group consisting of notes associated with radish, horseradish, wasabi, mustard, onion, garlic, leek, shallot, chive, and cress,
preferably selected from the group consisting of sulfur-containing aroma molecules with a molecular weight of less than 300 g/mol, preferably selected from the group consisting of thiols, thioethers, disulfides, trisulfides, thiophenes, thiocyanates, isothiocyanates, thionoesters, thioesters, dithiines, dithianes, dithiazines, pentathiepanes, tetrathianes, dithiolanes, trithiolanes, and S-oxides,
(B-3) aroma molecules found in meat or contributing to the aroma of meat, preferably selected from the group consisting of
C4-C12-pyrazines, C5-C12-pyridines, C4-C12-pyrroles, C4-C12-pyrrolines, C4-C10-oxazolines C4-C10-thiazoles, C4-C10-thiazolines, C3-C8-thiazolidines, C4-C12-furans, C4-C12-furanones, straight- or branched-chain alkanals having a total of 4 to 15 carbon atoms, straight- or branched-chain alkanols having a total of 5 to 10 carbon atoms, C4-C15-ketones, gamma-lactones having an even-number of total carbon atoms in the range of 6 to 12 carbon atoms, delta-lactones having an uneven-number of total carbon atoms in the range of 7 to 13 carbon atoms, C4-C8-alkanoic acids, C6-C15-hydrocarbons, and C16-C30-alkanoic acids having 0 to 5 carbon-carbon-double bonds, and C6-C12-phenols with 1 or 2 hydroxyl groups and 0 to 2 methoxy groups,
(B-4) aroma molecules imparting one or more sensorial impressions selected from the group consisting of thyme, clove, cinnamon, pimento, black pepper, white pepper, red chili, green chili, anise, lemongrass, vanilla, coconut, caramel, coriander, ginger, basil, sweet basil, tarragon, lemon, curry, fenugreek, and lime,
(B-5) amino acids, preferably selected from the group consisting of amino carboxylic acids and amino sulfonic acids, and the physiologically acceptable salts thereof,
(B-6) further flavor enhancers for meaty and/or roasty notes,
and
(B-7) substances selected from the group consisting of beef extract, soy sauce, soy sauce extract, soy protein, yeast extract, autolyzed yeast extract, malt extract, hydrolyzed vegetable protein, whey protein, caseinate, and barley extract.

8. Flavoring mixture according to claim 7, wherein constituent
(B-1) comprises or consists of substances selected from the group consisting of spilanthol, alpha-sanshool, alpha-hydroxysanshool, gamma-hydroxysanshool, gamma]hydroxysanshool), gamma-hydroxyisosanshool, gamma-dehydrosanshool, gamma-sanshool, bungeanool, isobungeanool, dihydrobungeanool, tetrahydrobungeanool, trans-pellitorine, cis-pellitorine, 2Z,4Z-decadienoic acid N-isobutylamide, 2Z,4E-decadienoic acid N-isobutylamide, 2E,4E-decadienoic acid N-([2S]-2-methylbutyl)amide, 2E,4E-decadienoic acid N-([2S]-2-methylbutyl)amide, 2E,4E-decadienoic acid N-([2R]-2-methylbutylamide), 2E,4Z-decadienoic acid N-(2-methylbutyl)amide, achilleamide, sarmentine, 2E-decenoic acid N-isobutylamide, 3E-decenoic acid N-isobutylamide, 3E-nonenoic acid N-isobutylamide, 2E,7Z,9E-undeca-2,7,9-trienoic acid N-isobutyl amide, 2E-undeca-2-en-8,10-diynoic acid N- isobutyl amide,
vanillyl alcohol n-propyl ether, vanillyl alcohol isopropyl ether, vanillyl alcohol n-butyl ether, vanillyl alcohol isobutyl ether, vanillyl alcohol isoamyl ether, vanillyl alcohol n-hexyl ether vanillyl alcohol methyl ether, vanillyl alcohol ethyl ether, gingerols, gingerdiones, shogaols, paradols, zingerone, capsaicin, dihydrocapsaicin, nordihydrocapsaicin, homocapsaicin, homodihydrocapsaicin, nonivamide,
chavicine, piperine, 1'-acetoxychavicol, 1'-acetoxychavicol acetate, polygodial, warburganal, muzigadial, merulidial, acetylmerulidial, aframodial, cinnamodial, cinnamolide, cinnamosmolide, capsicodendrin, velleral, isovelleral, isoisovelleral, isodrimeninol,
echinacea extract, jambu oleoresin, ginger oleoresin, ginger extracts, pepper extracts, black pepper extract, red pepper oleoresin, red pepper oil, Japanese pepper extracts, extracts of toothache grass (Ctenium aromaticum), extracts of tarragon (Artemisia dracunculus), pellitory root extracts (Anacyclus spp.), echinacea extracts (Echinaceae spp.), extracts of Szechuan pepper (Zanthoxylum spp.), Spilanthes or paracress extracts (Spilanthes spp.), extracts of Acmella spp., extracts of Achillea spp., extracts of Fagara species (Fagara zanthoxyloides), extracts of Heliopsis spp., extracts of Cissampelos glaberrima, extracts of Dinosperma erythrococca, bark extracts of Esenbeckia alata, extracts of Stauranthus perforatus, and extracts of water pepper (Persicaria hydropiper),
and/or
(B-2) comprises or consists of methanethiol, ethanthiol, propanethiol, isopropylthiol, allyl mercaptan, furfuryl mercaptan, 5-methyl-2-furfurylthiol, dimethyl sulfide, diallyl sulfide, methyl propyl sulfide, methyl isopropyl sulfide, dimethyl disulfide, diethyl sulfide, dipropyl disulfide, diallyl disulfide, allyl propyl disulfide, methyl isopropyl disulfide, methyl propyl disulfide, propyl propenyl disulfide, difurfuryl disulfide, furfuryl methyl disulfide, bis(2-methyl-3-furyl) disulfide, dimethyl trisulfide, diisopropyl trisulfide, dipropyl trisulfide, allyl methyl trisulfide, 2,3,4-trithiahexane, diallyl trisulfide, 2,3,5-trithiahexane, allyl isothiocyanate, phenylethyl isothiocyanate, cyclopropyl isothiocyanate, butyl isothiocyanate, 3-methylthiopropyl isothiocyanate, 4-hydroxybenzyl isothiocyanate, 4-methoxybenzyl isothiocyanate, sulforaphane, methional, 3-(methylthio) hexan-1-ol, 3-mercaptohexan-1-ol, 3-mercaptohexyl acetate, 3-mercaptohexyl butyrate, S-methyl thioacetate, 2-methylthiophene, 2-ethylthiophene, 2-propylthiophene, 2-butylthiophene, 2-hexylthiophene, 2-octylthiophene, 2,5-dimethylthiophene, 2-acetylthiophene, 3-(methylthiomethyl)thiophene, 2-thiophenyl methanethiol, 3-methyl-2-thiophenyl methanethiol, methyl-1,3-dithiolane, 3,5-dimethyl-1,2,4-trithiolane, allicin, ajoene, 3-[(prop-2-ene-1-sulfinyl)sulfanyl]prop-1-ene, 3-vinyl-(4*H*)-1,2-dithiin, 2-vinyl-(4*H*-1,3-dithiin, syn-propanethial S-oxide, syn-butanethial S-oxide, 2,4,6-trimethyl-perhydro-1,3,5-dithiazine, 2,4,6-trimethyldihydro-4H-1,3,5-dithiazine, 2,4,6-triethyldihydro-4H-1,3,5-dithiazine, 2-isopropyl-4,6-dimethyldihydro-1,3,5-dithiazine, 4-isopropyl-2,6-dimethyldihydro-1,3,5-dithiazine, 2,4,6-triisobutyl-5,6-dihydro-4H-1,3,5-dithiazine, 2-methyl-(3-methylthio)-furan, 2-methylfuran-3-thiol, 2-methyl-3-methylthiofuran, 2-methyltetrahydrofuran-3-thiol, 2,5-dimethylfuran-3-thiol, 2-methyl-3-(methyldisulfanyl)furan, 2-methyl-3-(methyl dithio)furan, 1,4-dithiane, 2,5-dihydroxy-1,4-dithiane, and 2,5-dihydroxy-2,5-dimethyl-1,4-dithiane,
and/or
(B-3) comprises or consists of aroma molecules selected from the group consisting of 2-acetyl-1-pyrroline, 2-propionylpyrroline, 2-acetylpyrrole, pyridine, 2-methylpyridine, 3-methylpyridine, 6-acetyl-2,3,4,5-tetrahydropyridine, 2-pentylpyridine, 2-(3-phenylpropyl)pyridine, furfural, 5-hydroxymethylfurfural, 2,5-dimethylfuran, 2-methyl-5-ethylfuran, 2-ethylfuran, 2-propylfuran, 2-butylfuran, 2-pentylfuran, 2-pentenylfuran, 2-acetylfuran, 4-hydroxy-5-methyl-3(2*H*-furanone, 2,5-dimethyl-4-hydroxy-(2*H*)furan-3-one, 4,5-dimethyl-3-hydroxy-2(5*H*)furanone, 2,4-dimethyl-3-oxazoline, 2,4-dimethyl-5-ethyl-3-oxazoline, 2,5-dimethyl-4-ethyl-3-oxazoline, 2,4,5-trimethyl-3-oxazoline, 2-acetyl-2-thiazoline, 2-propionyl-2-thiazoline, 2,4-dimethyl-3-thiazoline, 2,4,5-trimethylthiazoline, 2-isobutyl-4,5-dimethyl-3-thiazoline, 4-methylthiazole, 2-methylthiazole, 2-acetyl-2-thiazole, 2,4,5-trimethylthiazole, 2,4-dimethyl-5-ethylthiazole, 5-hydroxyethyl-4-methylthiazole, 1,3-benzothiazole, 1,3-thiazolidine, 2-methyl-1,3- thiazolidine, 2-isopropyl-1,3-thiazolidine,
2-methylpyrazine, 2-ethylpyrazine, 2-propylpyrazine, 2-isopropylpyrazine, 2-methoxypyrazine, 2,3-dimethylpyrazine, 2,5-dimethylpyrazine, 2,6-dimethylpyrazine, 2,3,5-trimethylpyrazine, 2,3,5,6-tetramethylpyrazine, 2-methyl-3-ethylpyrazine 2-ethyl-3,5-dimethylpyrazine, 2-ethyl-3,6-dimethylpyrazine, 3-ethyl-2,5-dimethylpyrazine, 2,3-diethyl-5-methylpyrazine, 3-isobutyl-2-methoxypyrazine, 3-isopropyl-2-methoxypyrazine, 2-acetylpyrazine, 2-acetyl-3-methylpyrazine, 5-acetyl-2-methylpyrazine, 6-acetyl-2-ethylpyrazine,
2-methylpropanal, 2-methybutanal, 3-methylbutanal, 4-pentenal, (E)-2-nonenal, hexanal, (E)-2-hexenal, 2,4-hexadienal, heptanal, 4-heptenal, octanal, (E)-2-octenal, octadienal, nonanal, (E,E)-2,4-nonadienal, (2E,6Z)-nonadienal, (E,E)-2,4-decadienal, (E,Z)-2,4-decadienal, (E)-2-undecenal, 12-methyl-tridecanal,
3-methyl-1-butanol, 1-pentanol, 1-penten-3-ol, 3-cis-hexenol, 3-methyl-2-hexen-1-ol, 1-heptanol, 3-octanol, 1-octene-3-ol,
3-hydroxy-2-butanone, 2-butanone, 2,3-butanedione, 2-pentanone, methyl isobutyl ketone, 1-pentene-3-one, 2-hexanone, oct-1-en-3-one, 2,3-octanedione, 2-nonanone, 2-decanone, 4-undecanone, 2-tridecanone, 3-methylcyclopentanone, 3-methylcyclopentenone, methyl cyclopentenolone, dimethyl cyclopentenolone, ethyl cyclopentenolone,
gamma-hexalactone, gamma-octalactone, gamma-decalactone, gamma-dodecalactone, delta-nonalactone,
butyric acid, isobutyric acid, isovaleric acid, pentanoic acid, hexanoic acid, isocaproic acid, heptanoic acid,
4-ethyl-1-methylhexane, 1,1,3-trimethylcyclohexane, naphthalene, alpha-pinene, beta-pinene, 3,6-dimethylundecane,
palmitic acid, stearic acid, linoleic acid, vaccenic acid, 11,14-eicosdienoic acid, arachidonic acid, 5,8,11,14,17-eicosapentaenoic acid,
phenol, o-cresol, m-cresol, p-cresol, guaiacol, 4-methylguaiacol, 4-ethylguaiacol, 4-propylguaiacol, 4-vinylguaiacol, 1,2-dihydroxybenzene, vanillin, eugenol, (E)-isoeugenol, (Z)-isoeugenol, acetovanillone, 4-(2-propio)-vanillone, 4-(1-propio)-vanillone, syringol, 4-methylsyringol, 4-ethylsyringol, 4-propylsyringol, syringaldehyde, 4-acetosyringol, 4-(2-propio)-syringone, 4-(1-propio)-syringone, 4-(2-propenyl)-syringol, (Z)-4-(1-propenyl)-syringol, (E)-4-(1-propenyl)-syringol, and 4-acetosyringone,
and/or
(B-4) comprises or consists of aroma molecules selected from the group consisting of
eugenol, cinnamaldehyde, anisole, anethole, estragole, thymol, carvacrol, vanillin, maltol, ethyl maltol, nerol, citral, citronellal, citronellol, nerolidol, linalool, massoia lactone, sotolon, caryophyllene, alpha-phellandrene, terpinene-4-ol, and gamma-nonalactone,
and/or
(B-5) comprises or consists of amino acids selected from the group consisting of arginine, alanine, valine, isoleucine, leucine, methionine, phenylalanine, tyrosine, tryptophan, serine, asparagine, aspartic acid, threonine, cysteine, proline, 4-hydroxyproline, glutamine, lysine, 5-hydroxylysine, histidine, anserine, carnosine, S-allylmercaptocysteine, S-allylcysteine, alliin, taurine, gamma-amino acids, and the physiologically acceptable salts thereof,
and/or
(B-6) comprises or consists of the group consisting of sodium lactate, potassium lactate, sodium chloride, potassium chloride, hexametaphosphate salts, sodium and/or potassium salts of tripolyphosphate, tetrasodium pyrophosphate, monosodium glutamate, inosine monophosphate, disodium inosinate, guanosine monophosphate, and disodium guanylate.

9. Flavoring mixture according to any of claims 6 to 8, comprising one, two, three or more additional ingredients selected from the group consisting of
(B-8) solid carrier substances selected from the group consisting of chemically modified starches, degraded starches, pectins, glutens, starch hydrolysates, chemically modified celluloses, gums, proteins, silicon dioxide, cyclodextrins, and milk powders,
and/or
(B-9) food acids, preferably selected from the group consisting of acetic acid, adipic acid, caffeotannic acid, citric acid, iso-citric acid, fumaric acid, galacturonic acid, glucuronic acid, glyceric acid, glycolic acid, lactic acid, malic acid, oxalic acid, pyruvic acid, quinic acid, shikimic acid, succinic acid, tannic acid, ascorbic acid and tartaric acid, and the physiologically acceptable salts thereof.

10. Flavoring mixture as defined in any one of claims 6 to 9 imparting one or more sensory impressions selected from the group of meat, seafood, fish, seaweed, mushrooms, and *Allium* species, preferably selected from the group consisting of onion, garlic, leek, shallot, beef, pork, chicken, turkey, duck, goat, lamb, mutton, deer, springbok, catfish, tuna, cod, eel, sword fish, hake, salmon, trout, tilapia, whiting, mussel, scallop, clam shell, shrimp, prawn, squid, crab, button mushroom, brown mushroom, black ear mushroom, shiitake mushroom, chanterelle mushroom, chestnut mushroom, clamshell mushroom, cloud ear mushroom, enoki mushromms, eryngii mushroom, gamboni mushroom, maitake mushroom, matsutake mushroom, morels, nameko mushroom, oyster mushroom, porcini mushroom, portobello mushroom, puff ball mushroom, and straw mushroom.

11. Composition for oral consumption, comprising
(a) a sensorially effective amount of
- a compound or of a mixture as defined in claims 1 to 4,
or
- a flavoring mixture as defined in any one of claims 6 to 10,
and
(b) one or more further constituents suitable for oral consumption.

12. Composition as claimed in claim 11, wherein the further constituents suitable for consumption are:
- one or more emulsifiers, preferably selected from the group consisting of lecithins, phospholipids, monoacylglycerols, and diacylglycerols,
and/or
- one or more vegetable fatty oils, preferably selected from the group consisting of soy oil, olive oil, sunflower oil, safflower oil, corn oil, coconut oil, peanut oil, hazelnut oil, almond oil, walnut oil, palm kernel oil, pumpkin seed oil, canola oil, sesame oil, cottonseed oil, wheat germ oil, linseed oil, and rice bran oil,
and/or
- one or more preservatives, preferably selected from the group consisting of benzoic acid, sorbic acid, phosphoric acid, and the physiologically acceptable salts of these acids,
and/or
- one or more antioxidants and optionally one or more substances for intensifying the antioxidative effect of said antioxidants,
and/or
- one or more vitamins and the physiologically acceptable salts or esters thereof,
and/or
- one or more essential oils, preferably selected from the group consisting of aniseed oil, basil oil, bergamot oil, curry leaf oil, winter savory oil, summer savory oil, bucco-leaf oil, cardamom oil, lemon oil, coriander oil, costus root oil, cumin oil, dill oil, dillseed oil, tarragon oil, fennel oil, asafoetida oil, ginger oil, garlic oil, onion oil, leek oil, shallot oil, carrot-seed oil, caraway oil, lemongrass oil, distilled lime oil, pressed lime oil, bay-leaf oil, mace oil, marjoram oil, nutmeg oil, myrtle oil, clove leaf oil, clove flower oil, neroli oil, orange oil, origanum oil, parsley leaf oil, parsley seed oil, pepper oil, pimento oil, pine oil, rosemary oil, celery seed oil, tagetes oil, tea-tree oil, thyme oil, verbena oil, juniper oil, hyssop oil, cinnamon leaf oil, cinnamon bark oil,
and/or
- one or more coloring agents.

13. Ready-to-eat product, ready-to-use product, or semifinished product for oral use or oral consumption, comprising a sensorially effective amount of
- a compound or mixture as defined in claims 1 to 4, or
- a flavoring mixture as defined in any one of claims 6 to 10, or
- a composition for oral consumption a defined in claim 11 or 12,
and one or more further ingredients for oral consumption selected from the group consisting of base or bulk materials, additives, excipients, and functional ingredients.

14. Ready-to-eat or ready-to-use product according to claim 13, comprising
- a total amount of the compounds of Formula (I) as defined in claims 1 to 4 in the range of from 1 ppm to 1000 ppm, preferably of from 2 ppm to 750 ppm,
and preferably no sodium glutamate or a content of from 0.00001 to 2% by weight, preferably 0.001 to 1% by weight, more preferably 0.01 % by weight to 0.25%, in particular 0.025% by weight to 0.1% by weight of sodium glutamate,
in each case based on the total weight of the product.

15. Semifinished product according to claim 13 comprising
- a total amount of the compounds of Formula (I) as defined in claims 1 to 4 in the range of from 50 ppm to 50000 ppm, preferably of 75 ppm to 25000 ppm,
and preferably no sodium glutamate or a content of from 0.001 to 10% by weight, preferably 0.01 to 5% by weight, in particular 0.1% by weight to 2% by weight of sodium glutamate,
in each case based on the total weight of the semifinished product.

16. Method for imparting or intensifying a sensory effect, in particular for
(a) imparting or intensifying an umami flavor
and/or
(b) intensifying one, several or all of the sensory impressions mouthfeel, meaty, roasty, salty, mouth-watering and long-lastingness of other orally consumable constituents, preferably of other orally consumable constituents of (i) a ready-to-use or ready-to-eat product or (ii) a semifinished product for oral use or oral consumption, as defined in any one of claims 13 to 15,
including the following step:
1.) mixing a sensorially effective amount of a compound or mixture as defined in claims 1 to 4, a flavoring mixture according to any one of claims 7 to 10, or a composition for oral consumption according to claims 11 to 12, with one or more further orally consumable constituents, preferably one or more further orally consumable constituents of (i) a ready-to-eat or ready-to-use product or (ii) a semifinished product, preferably one or more further orally consumable constituents of (i) a ready-to-eat or ready-to-use product or (ii) a semifinished product as defined in any one of claims 13 to 15,
or
applying a sensorially effective amount of a compound or mixture as defined in claims 1 to 4, a flavoring mixture according to any one of claims 7 to 10, or a composition for oral consumption according to claim 11 or 12, to (i) a ready-to-eat or ready-to-use product or (ii) a semifinished product, preferably (i) a ready-to-eat or ready-to-use product or (ii) a semifinished product as defined in any one of claims 13 to 15.
